# EUROPEAN PATENT APPLICATION

(11) **EP 3 101 140 A1**
(43) Date of publication of application: **07.12.2016**
(21) Application number: 16155147.8
(22) Date of filing: 11.02.2016
(51) Int. Cl.: C12Q 1/68

(54) **GENETIC TESTING FOR PREDICTING RESISTANCE OF SHIGELLA SPECIES AGAINST ANTIMICROBIAL AGENTS**

(30) Priority: 02.06.2015 WO PCT/EP2015/062202
(71) Applicant: Siemens Healthcare GmbH, 91052 Erlangen (DE)
(72) Inventor: Keller, Andreas, 66125 Dudweiler (DE); Schmolke, Susanne, 91052 Erlangen (DE); Stähler, Cord Friedrich, 69493 Hirschberg an der Bergstraße (DE); Backes, Christina, 66125 Saarbrücken (DE)

(57) **Abstract**

The invention relates to a method of determining an infection of a patient with Shigella species potentially resistant to antimicrobial drug treatment, a method of selecting a treatment of a patient suffering from an antibiotic resistant Shigella infection, and a method of determining an antibiotic resistance profile for bacterial microorganisms of Shigella species, as well as computer program products used in these methods. In an exemplary method, a sample 1, is used for molecular testing 2, and then a molecular fingerprint 3 is taken. The result is then compared to a reference library 4, and the result 5 is reported.

## Description

The present invention relates to a method of determining an infection of a patient with Shigella species potentially resistant to antimicrobial drug treatment, a method of selecting a treatment of a patient suffering from an infection with a potentially resistant Shigella strain, and a method of determining an antimicrobial drug, e.g. antibiotic, resistance profile for bacterial microorganisms of Shigella species, as well as computer program products used in these methods.

Antibiotic resistance is a form of drug resistance whereby a sub-population of a microorganism, e.g. a strain of a bacterial species, can survive and multiply despite exposure to an antibiotic drug. It is a serious and health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. According to the World Health Organization's antimicrobial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%. Shigella species are Gram-negative, facultative anaerobic rods belonging to the family of Enterobacteriaceae. The genus is divided into four serogroups with multiple serotypes: A (S dysenteriae); B (S flexneri); C (S boydii); and D (S sonnei). Shigella species are a major cause of diarrhoea and dysentery (diarrhoea with blood and mucus in the stools) throughout the world. The susceptibility of Shigella species to various antibiotics (e.g. ampicillin, trimetropin-sulfamethoxazole) decreased significantly from the 1970-80 to the 1990 - 2000.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:
1) Efflux pumps are high-affinity reverse transport systems located in the membrane that transports the antibiotic out of the cell, e.g. resistance to tetracycline.
2) Specific enzymes modify the antibiotic in a way that it loses its activity. In the case of streptomycin, the antibiotic is chemically modified so that it will no longer bind to the ribosome to block protein synthesis.
3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation.

Generally, testing for susceptibility/resistance to antimicrobial agents is performed by culturing organisms in different concentration of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days during which the patient is treated empirically. A significant reduction of time-to-result is needed especially in patients with life-threatening disease and to overcome the widespread misuse of antibiotics.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

For some drugs such it is known that at least two targets are addressed, e.g. in case of Ciprofloxacin (drug bank ID 00537; http://www.drugbank.ca/drugs/DB00537) targets include DNA Topoisomerase IV, DNA Topoisomerase II and DNA Gyrase. It can be expected that this is also the case for other drugs although the respective secondary targets have not been identified yet. In case of a common regulation, both relevant genetic sites would naturally show a co-correlation or redundancy.

It is known that drug resistance can be associated with genetic polymorphisms. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):523) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al (Chewapreecha et al (2014) Comprehensive Identification of single nucleotid polymorphisms associated with beta-lactam resistance within pneumococcal mosaic genes. PLoS Genet 10(8): e1004547) used a comparable approach to identify mutations in gram-positive Streptococcus Pneumonia.

The fast and accurate detection of infections with Shigella species and the prediction of response to anti-microbial therapy represent a high unmet clinical need.
This need is addressed by the present invention.

### Summary of the Invention

The present inventors addressed this need by carrying out whole genome sequencing of a large cohort of Shigella clinical isolates and comparing the genetic mutation profile to classical culture based antimicrobial susceptibility testing with the goal to develop a test which can be used to detect bacterial susceptibility/resistance against antimicrobial drugs using molecular testing.

The inventors performed extensive studies on the genome of bacteria of Shigella species either susceptible or resistant to antimicrobial, e.g. antibiotic, drugs. Based on this information, it is now possible to provide a detailed analysis on the resistance pattern of Shigella strains based on individual genes or mutations on a nucleotide level. This analysis involves the identification of a resistance against individual antimicrobial, e.g. antibiotic, drugs as well as clusters of them. This allows not only for the determination of a resistance to a single antimicrobial, e.g. antibiotic, drug, but also to groups of antimicrobial drugs, e.g. antibiotics such as lactam or quinolone antibiotics, or even to all relevant antibiotic drugs.

Therefore, the present invention will considerably facilitate the selection of an appropriate antimicrobial, e.g. antibiotic, drug for the treatment of a Shigella infection in a patient and thus will largely improve the quality of diagnosis and treatment.

According to a first aspect, the present invention discloses a diagnostic method of determining an infection of a patient with Shigella species potentially resistant to antimicrobial drug treatment, which can be also described as a method of determining an antimicrobial drug, e.g. antibiotic, resistant Shigella infection of a patient, comprising the steps of:
a) obtaining or providing a sample containing or suspected of containing at least one Shigella species from the patient;
b) determining the presence of at least one mutation in at least two genes from the group of genes listed in Table 1 or Table 2 below, wherein the presence of said at least two mutations is indicative of an infection with an antimicrobial drug resistant, e.g. antibiotic resistant, Shigella strain in said patient.

An infection of a patient with Shigella species potentially resistant to antimicrobial drug treatment herein means an infection of a patient with Shigella species wherein it is unclear if the Shigella species is susceptible to treatment with a specific antimicrobial drug or if it is resistant to the antimicrobial drug.

In step b) above, as well as corresponding steps, at least one mutation in at least two genes is determined, so that in total at least two mutations are determined, wherein the two mutations are in different genes.

**Table 1: List of genes**

| | | | | |
|---|---|---|---|---|
| SSON53_12105 | SSON53_12455 | SSON53_12070 | SSON53_12475 | metH |
| SSON53_00425 | SSON53_23830 | SSON53_13575 | flgE | SSON53_12500 |
| SSON53_06660 | SSON53_24790 | SSON53_16770 | SSON53_24480 | SSON53_08770 |
| SSON53_04795 | SSON53_07430 | SSON53_14085 | SSON53_07440 | SSON53_08950 |
| SSON53_25555 | SSON53_09145 | SSON53_25565 | SSON53_24780 | SSON53_04400 |
| SSON53_15280 | rimO | SSON53_04930 | SSON53_23390 | rhaB |
| SSON53_17960 | thiH | SSON53_03725 | SSON53_09500 | SSON53_25405 |
| SSON53_13530 | astD | SSON53_07945 | SSON53_10080 | SSON53_10655 |
| SSON53_13400 | SSON53_13555 | SSON53_14945 | SSON53_15285 | fucI |
| SSON53_02755 | SSON53_06415 | SSON53_04615 | SSON53_17330 | pdxA |

**Table 2: List of genes**

| | | | | |
|---|---|---|---|---|
| SSON53_12105 | SSON53_12455 | SSON53_12475 | metH | SSON53_00425 |
| SSON53_23830 | SSON53_13575 | flgE | SSON53_12500 | SSON53_24790 |
| SSON53_16770 | SSON53_08770 | SSON53_04795 | SSON53_07430 | SSON53_14085 |
| SSON53_07440 | SSON53_08950 | SSON53_25555 | SSON53_09145 | SSON53_25565 |
| SSON53_24780 | SSON53_04400 | SSON53_15280 | SSON53_04930 | SSON53_23390 |
| rhaB | SSON53_17960 | thiH | SSON53_03725 | SSON53_09500 |
| SSON53_25405 | SSON53_13530 | astD | SSON53_07945 | SSON53_10080 |
| SSON53_10655 | SSON53_13400 | SSON53_13555 | SSON53_14945 | SSON53_15285 |
| fucI | SSON53_02755 | SSON53_06415 | SSON53_04615 | SSON53_17330 |
| pdxA | SSON53_04690 | SSON53_04945 | SSON53_06005 | SSON53_06085 |

According to a second aspect, the present invention relates to a method of selecting a treatment of a patient suffering from an infection with a potentially resistant Shigella stain, e.g. from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least two genes from the group of genes listed in Table 1 or Table 2 above, wherein the presence of said at least two mutations is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

A third aspect of the present invention relates to a method of determining an antimicrobial drug, e.g. antibiotic, resistance profile for bacterial microorganisms of Shigella species, comprising:
obtaining or providing a first data set of gene sequences of a plurality of clinical isolates of Shigella species;
providing a second data set of antimicrobial drug, e.g. antibiotic, resistance of the plurality of clinical isolates of Shigella species;
aligning the gene sequences of the first data set to at least one, preferably one, reference genome of Shigella, or assembling the gene sequence of the first data set, at least in part;
analyzing the gene sequences of the first data set for genetic variants to obtain a third data set of genetic variants;
correlating the third data set with the second data set and statistically analyzing the correlation; and
determining the genetic sites in the genome of Shigella associated with antimicrobial drug, e.g. antibiotic, resistance.

In addition, the present invention relates in a fourth aspect to a method of determining an antimicrobial drug, e.g. antibiotic, resistance profile for a bacterial microorganism belonging to the species Shigella comprising the steps of
a) obtaining or providing a sample containing or suspected of containing the bacterial microorganism;
b) determining the presence of a mutation in at least one gene of the bacterial microorganism as determined by the method according to the third aspect of the present invention;
wherein the presence of a mutation is indicative of a resistance to an antimicrobial, e.g. antibiotic, drug.

Furthermore, the present invention discloses in a fifth aspect a diagnostic method of determining an infection of a patient with Shigella species potentially resistant to antimicrobial drug treatment, which can, like in the first aspect, also be described as method of determining an antimicrobial drug, e.g. antibiotic, resistant Shigella infection of a patient, comprising the steps of:
a) obtaining or providing a sample containing or suspected of containing a bacterial microorganism belonging to the species Shigella from the patient;
b) determining the presence of at least one mutation in at least one gene of the bacterial microorganism belonging to the species Shigella as determined by the method according to the third aspect of the present invention, wherein the presence of said at least one mutation is indicative of an antimicrobial drug, e.g. antibiotic, resistant Shigella infection in said patient.

Also disclosed is in a sixth aspect a method of selecting a treatment of a patient suffering from an infection with a potentially resistant Shigella strain, e.g. from an antimicrobial drug, e.g. antibiotic, resistant Shigella infection, comprising the steps of:
a) obtaining or providing a sample containing or suspected of containing a bacterial microorganism belonging to the species Shigella from the patient;
b) determining the presence of at least one mutation in at least one gene of the bacterial microorganism belonging to the species Shigella as determined by the method according to the third aspect of the present invention, wherein the presence of said at least one mutation is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

A seventh aspect of the present invention relates to a method of acquiring, respectively determining, an antimicrobial drug, e.g. antibiotic, resistance profile for a bacterial microorganisms of Shigella species, comprising:
obtaining or providing a first data set of gene sequences of a clinical isolate of Shigella species;
providing a second data set of antimicrobial drug, e.g. antibiotic, resistance of a plurality of clinical isolates of Shigella species;
aligning the gene sequences of the first data set to at least one, preferably one, reference genome of Shigella, or assembling the gene sequence of the first data set, at least in part;
analyzing the gene sequences of the first data set for genetic variants to obtain a third data set of genetic variants of the first data set;
correlating the third data set with the second data set and statistically analyzing the correlation; and
determining the genetic sites in the genome of Shigella of the first data set associated with antimicrobial drug, e.g. antibiotic, resistance.

According to an eighth aspect, the present invention discloses a computer program product comprising executable instructions which, when executed, perform a method according to the fifth, sixth or seventh aspect of the present invention.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

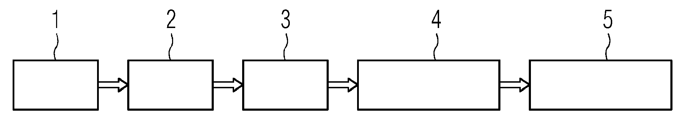

Fig. 1 shows schematically a read-out concept for a diagnostic test according to a method of the present invention.

### Detailed description of the present invention

### Definitions

Unless defined otherwise, technical and scientific terms used herein have the same meaning as commonly understood by one of ordinary skill in the art to which this invention belongs.

An "antimicrobial drug" in the present invention refers to a group of drugs that includes antibiotics, antifungals, antiprotozoals, and antivirals. According to certain embodiments, the antimicrobial drug is an antibiotic.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to an information which can be derived from the sequence of a nucleic acid molecule, such as the sequence itself or a variation in the sequence as compared to a reference sequence.

The term "mutation" relates to a variation in the sequence as compared to a reference sequence. Such a reference sequence can be a sequence determined in a predominant wild type organism or a reference organism, e.g. a defined and known bacterial strain or substrain. A mutation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, and, in particular, a single nucleotide polymorphism (SNP).

In the context of the present invention a "sample" is a sample which comprises at least one nucleic acid molecule from a bacterial microorganism. Examples for samples are: cells, tissue, body fluids, biopsy specimens, blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to high-throughput sequencing technologies that parallelize the sequencing process, producing thousands or millions of sequences at once. Examples include Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain aspects, it refers to one or more Shigella species.

A reference to a microorganism or microorganisms in the present description comprises a reference to one microorganism as well a plurality of microorganisms, e.g. two, three, four, five, six or more microorganisms.

A vertebrate within the present invention refers to animals having a vertebrae, which includes mammals - including humans, birds, reptiles, amphibians and fishes. The present invention thus is not only suitable for human medicine, but also for veterinary medicine.

According to certain embodiments, the patient in the present methods is a vertebrate, more preferably a mammal and most preferred a human patient.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005 might be used as a guideline. Regarding the formulation of a ready-to-use medicament, reference is made to "Remington, The Science and Practice of Pharmacy", 22nd edition, 2013.

Assembling of a gene sequence can be carried out by any known method and is not particularly limited.

According to certain embodiments, mutations that were found using alignments can also be compared or matched with alignment-free methods, e.g. for detecting single base exchanges, for example based on contigs that were found by assemblies. For example, reads obtained from sequencing can be assembled to contigs and the contigs can be compared to each other.

According to a first aspect, the present invention relates to a diagnostic method of determining an infection of a patient with Shigella species potentially resistant to antimicrobial drug treatment, which can also be described as method of determining an antimicrobial drug, e.g. antibiotic, resistant Shigella infection of a patient, comprising the steps of:
a) obtaining or providing a sample containing or suspected of containing at least one Shigella species from the patient;
b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53 23390, rhaB, SSON53 17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of an infection with an antimicrobial, e.g. antibiotic, resistant Shigella strain in said patient.

In this method, as well as the other methods of the invention, the sample can be provided or obtained in any way, preferably non-invasive, and can be e.g. provided as an *in vitro* sample or prepared as *in vitro* sample.

According to certain aspects, mutations in at least two, three, four, five, six, seven, eight, nine or ten genes are determined in any of the methods of the present invention, e.g. in at least two genes or in at least three genes. Instead of testing only single genes or mutants, a combination of several variant positions can improve the prediction accuracy and further reduce false positive findings that are influenced by other factors. Therefore, it is in particular preferred to determine the presence of a mutation in 2, 3, 4, 5, 6, 7, 8 or 9 (or more) genes selected from Table 1 or 2.

For the above genes, i.e. the genes also denoted in Tables 1 and 2, the highest probability of a resistance to at least one antimicrobial drug, e.g. antibiotic, could be observed, with p-values smaller than 10⁻³⁰, particularly smaller than 10⁻⁴⁰, indicating the high significance of the values (n= 470; α = 0.05). Details regarding Tables 1 and 2 can be taken from Tables 3 and 4 (4a, 4b, 4c) disclosed in the Examples. Having at least two genes with mutations determined, a high probability of an antimicrobial drug, e.g. antibiotic, resistance could be determined. The genes in Table 1 thereby represent the 50 best genes for which a mutation was observed in the genomes of Shigella species, whereas the genes in Table 2 represent the 50 best genes for which a cross-correlation could be observed for the antimicrobial drug, e.g. antibiotic, susceptibility testing for Shigella species as described below.

According to certain embodiments, the obtaining or providing a sample containing or suspected of containing at least one Shigella species from the patient in this method - as well as the other methods of the invention - can comprise the following:
A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly of at least one Shigella species. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be used to identify the nucleic acids, and thus genes, of the microorganism, e.g. of Shigella species, to be identified, by known methods, e.g. fingerprinting methods, comparing genomes and/or aligning to at least one, or more, genomes of one or more species of the microorganism of interest, i.e. a reference genome, etc., forming a third data set of aligned genes for a Shigella species - discarding additional data from other sources, e.g. the vertebrate. Reference genomes are not particularly limited and can be taken from several databases. Depending on the microorganism, different reference genomes or more than one reference genomes can be used for aligning. Using the reference genome - as well as also the data from the genomes of the other species, e.g. Shigella species - mutations in the genes for each species and for the whole multitude of samples of different species, e.g. Shigella species, can be obtained.

For example, it is useful in genome-wide association studies to reference the points of interest, e.g. mutations, to one constant reference for enhanced standardization. In case of the human with a high consistency of the genome and 99% identical sequences among individuals this is easy and represents the standard, as corresponding reference genomes are available in databases. In case of organisms that trigger infectious diseases (e.g. bacteria and viruses) this is much more difficult, though. One possibility is to fall back on a virtual pan genome which contains all sequences of a certain genus. A further possibility is the analysis of all available references, which is much more complex. Therein all *n* references from a database (e.g. RefSeq) are extracted and compared with the newly sequenced bacterial genomes *k*. After this, matrices (% of mapped reads, % of covered genome) are applied to estimate which reference is best suited to all new bacteria. However, n x *k* complete alignments are carried out. Having a big number of references, though, stable results can be obtained, as is the case for Shigella.

According to certain embodiments, the genomes of Shigella species are referenced to one reference genome. However, it is not excluded that for other microorganisms more than one reference genome is used. In the present methods, the reference genome of Shigella is NC_016822 as annotated at the NCBI according to certain embodiments.

The reference sequence was obtained from Shigella strain NC_016822 (http://www.genome.jp/dbget-bin/www_bget?refseq+NC_016822)
LOCUS NC_016822 4988504 bp DNA circular CON 07-FEB-2015 DEFINITION Shigella sonnei 53G main chromosome, complete genome.
ACCESSION NC_016822
VERSION NC_016822.1 GI:377520096
DBLINK BioProject: PRJNA224116
   Assembly: GCF_000283715.1
KEYWORDS RefSeq; complete genome; complete replicon.
SOURCE Shigella sonnei 53G
ORGANISM Shigella sonnei 53G
Bacteria; Proteobacteria; Gammaproteobacteria; Enterobacteriales;
Enterobacteriaceae; Shigella. REFERENCE 1
AUTHORS Thomson,N.R.
TITLE The genome of Shigella sonnei strain 53G
JOURNAL Unpublished
REFERENCE 2 (bases 1 to 4988504)
AUTHORS Aslett,M.A.
TITLE Direct Submission
JOURNAL Submitted (30-NOV-2011) Aslett M.A., Wellcome Trust Sanger
Institute, Pathogen Sequencing Unit, Wellcome Trust Genome Campus,
Hinxton, Cambridge, Cambridgeshire CB10 1SA, UNITED KINGDOM

Alternatively or in addition, the gene sequence of the first data set can be assembled, at least in part, with known methods, e.g. by de-novo assembly or mapping assembly. The sequence assembly is not particularly limited, and any known genome assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. Shigella species, can be removed after the nucleic acids of interest are identified, e.g. by filtering the data out. Such data can e.g. include nucleic acids of the patient, e.g. the vertebrate, e.g. human, and/or other microorganisms, etc. This can be done by e.g. computational subtraction, as developed by Meyerson et al. 2002. For this, also aligning to the genome of the vertebrate, etc., is possible. For aligning, several alignment-tools are available. This way the original data amount from the sample can be drastically reduced.

Also after such removal of "excess" data, fingerprinting and/or aligning, and/or assembly, etc. can be carried out, as described above, forming a third data set of aligned or assembled genes for a Shigella species.

Using these techniques, genes with mutations of the microorganism of interest, e.g. Shigella species, can be obtained for various species.

When testing these same species for antimicrobial drug, e.g. antibiotic, susceptibility of a number of antimicrobial drugs, e.g. antibiotics, e.g. using standard culturing methods on dishes with antimicrobial drug, e.g. antibiotic, intake, as e.g. described below, the results of these antimicrobial drug, e.g. antibiotic, susceptibility tests can then be cross-referenced/correlated with the mutations in the genome of the respective microorganism, e.g. Shigella. Using several, e.g. 50 or more than 50, 100 or more than 100, 200 or more than 200, 300 or more than 300, 400 or more than 400, or 450 or more than 450 different species of a microorganism, e.g. different Shigella species, statistical analysis can be carried out on the obtained cross-referenced data between mutations and antimicrobial drug, e.g. antibiotic, susceptibility for these number of species, using known methods.

Regarding culturing methods, samples can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Correlation of the nucleic acid / gene mutations with antimicrobial drug, e.g. antibiotic, resistance can be carried out in a usual way and is not particularly limited. For example, resistances can be correlated to certain genes or certain mutations, e.g. SNPs, in genes. After correlation, statistical analysis can be carried out.

In addition, statistical analysis of the correlation of the gene mutations with antimicrobial drug, e.g. antibiotic, resistance is not particularly limited and can be carried out, depending on e.g. the amount of data, in different ways, for example using analysis of variance (ANOVA) or Student's t-test, for example with a sample size n of 50, 100, 200, 300, 400 or 450, and a level of significance (α-error-level) of e.g. 0.05 or smaller, e.g. 0.05, preferably 0.01 or smaller. A statistical value can be obtained for each gene and/or each position in the genome as well as for all antibiotics tested, a group of antibiotics or a single antibiotic. The obtained p-values can also be adapted for statistical errors, if needed.

For statistically sound results a multitude of individuals should be sampled, with n = 50, 100, 200, 300, 400 or 450, and a level of significance (α-error-level) of e.g. 0.05 or smaller, e.g. 0.05, preferably 0.01 or smaller. According to certain embodiments, particularly significant results can be obtained for n = 200, 200, 400 or 450.

After the above procedure has been carried out for more than 450, e.g. 470, individual species of Shigella, the data disclosed in Tables 1 and 2 were obtained for the statistically best correlations between gene mutations and antimicrobial drug, e.g. antibiotic, resistances. Thus, mutations in these genes were proven as valid markers for antimicrobial drug, e.g. antibiotic, resistance.

According to a further aspect, the present invention relates in a second aspect to a method of selecting a treatment of a patient suffering from an infection with a potentially resistant Shigella stain, e.g. from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_044795, SSON53_07430, SSON53_14085, SSON53_07440, SSON53_08950, SSON53_25555, SSON53_09145, SSON53 25565, SSON53 24780, SSON53 04400, SSON53_15280, rimO, SSON53 04930, SSON53 23390, rhaB, SSON53 17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53 23390, rhaB, SSON53 17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

In this method, the steps a) of obtaining or providing a sample and b) of determining the presence of at least one mutation are as in the method of the first aspect.

The identification of the at least one or more antimicrobial, e.g. antibiotic, drug in step c) is then based on the results obtained in step b) and corresponds to the antimicrobial, e.g. antibiotic, drug(s) that correlate(s) with the mutations. Once these antimicrobial drugs, e.g. antibiotics, are ruled out, the remaining antimicrobial drugs, e.g. antibiotic drugs/antibiotics, can be selected in step d) as being suitable for treatment.

In the description, references to the first and second embodiment also apply to the 19^{th}, 20^{th}, 21^{st} and 22^{nd} embodiment, referring to the same genes, unless clear from the context that they don't apply.

According to certain embodiments, the antimicrobial drug, e.g. antibiotic, in the method of the first or second aspect, as well as in the other methods of the invention, is at least one selected from the group of β-lactams, β-lactam inhibitors, quinolines and derivatives thereof, aminoglycosides, polyketides, respectively tetracyclines, and folate synthesis inhibitors.

In the methods of the invention the resistance of Shigella to one or more antimicrobial, e.g. antibiotic, drugs can be determined according to certain embodiments.

Results with particular significance and low p-values can be obtained in the methods of the first and second aspect when the antimicrobial, e.g. antibiotic, drug is selected from lactam antibiotics, and particularly when the presence of a mutation in the following genes is determined: SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, and/or pdxA, or SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, SSON53 04930, SSON53 23390, rhaB, SSON53 17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and/or SSON53_06085.

For the lactam antibiotics, the p-values are that low for these genes that a statistically significant determination of antibiotic susceptibility is possible in particular.

According to certain embodiments of the first and/or second aspect of the invention the antimicrobial, e.g. antibiotic, drug is selected from polyketide antibiotics, preferably tetracycline antibiotics, and the presence of a mutation in the following genes is determined: metH, SSON53_24790, SSON53 08770, SSON53 24780, SSON53 15280, SSON53_10655, SSON53 13555, and/or SSON53 06415, or metH, SSON53_24790, SSON53 08770, SSON53 24780, SSON53 15280, SSON53_10655, SSON53_13555, and/or SSON53_06415. According to certain embodiments, the antimicrobial drug is an antibiotic/antibiotic drug.

According to certain embodiments of the first and/or second aspect of the invention, determining the nucleic acid sequence information or the presence of a mutation comprises determining the presence of a single nucleotide at a single position in a gene. Thus the invention comprises methods wherein the presence of a single nucleotide polymorphism or mutation at a single nucleotide position is detected.

According to certain embodiments, the antibiotic drug in the methods of the present invention is selected from the group consisting of Amoxicillin/K Clavulanate (AUG), Ampicillin (AM), Aztreonam (AZT), Cefazolin (CFZ), Cefepime (CPM), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).

The inventors have surprisingly found that mutations in certain genes are indicative not only for a resistance to one single antimicrobial, e.g. antibiotic, drug, but to groups containing several drugs.

According to certain embodiments of the first and/or second aspect of the invention, the gene is from Table 1, the antibiotic drug is selected from lactam antibiotics and a mutation in at least one of the following genes is detected with regard to reference genome NC_016822: SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA.

According to certain embodiments of the first and/or second aspect of the invention, the gene is from Table 1, the antibiotic drug is selected from polyketide, preferably tetracycline antibiotics and a mutation in at least one of the following genes is detected with regard to reference genome NC_016822: metH, SSON53_24790, SSON53_08770, SSON53_24780, SSON53_15280, SSON53_10655, SSON53_13555, SSON53_06415.

According to certain embodiments of the first and/or second aspect of the invention, the gene is from Table 2, the antibiotic drug is selected from lactam antibiotics and a mutation in at least one of the following genes is detected with regard to reference genome NC_016822: SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, SSON53_06085.

According to certain embodiments of the first and/or second aspect of the invention, the gene is from Table 2, the antibiotic drug is selected from polyketide, preferably tetracycline antibiotics and a mutation in at least one of the following genes is detected with regard to reference genome NC_016822: metH, SSON53_24790, SSON53_08770, SSON53_24780, SSON53_15280, SSON53_10655, SSON53_13555, SSON53_06415.

For specific antimicrobial drugs, e.g. antibiotics, specific positions in the above genes can be determined where a high statistical significance is observed. The inventors found that, apart from the above genes indicative of a resistance against antibiotics, also single nucleotide polymorphisms (= SNP's) may have a high significance for the presence of a resistance against defined antibiotic drugs. The analysis of these polymorphisms on a nucleotide level may further improve and accelerate the determination of a drug resistance to antimicrobial drugs, e.g. antibiotics, in Shigella.

According to certain embodiments of the first and/or second aspect of the invention, the gene is from Table 1, the antibiotic drug is selected from lactam antibiotics and a mutation in at least one of the following nucleotide positions is detected with regard to reference genome NC_016822: 2241215, 2309519, 2233949, 2314492, 4595667, 87512, 4502174, 2543074, 1159191, 2325584, 1303652, 4707429, 3134050, 4648709, 1655745, 949314, 1427818, 2636621, 1429397, 1690889, 4856482, 1723070, 4857482, 4705326, 862961, 2876708, 887353, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014.

According to certain embodiments of the first and/or second aspect of the invention, the gene is from Table 1, the antibiotic drug is selected from polyketide, preferably tetracycline antibiotics and a mutation in at least one of the following nucleotide positions is detected with regard to reference genome NC_016822: 4595667, 4707429, 1655745, 4705326, 2876708, 1990704, 2539010, 1257335.

According to certain embodiments of the first and/or second aspect of the invention, the gene is from Table 2, the antibiotic drug is selected from lactam antibiotics and a mutation in at least one of the following nucleotide positions is detected with regard to reference genome NC_016822: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the gene is from Table 2, the antibiotic drug is selected from polyketide, preferably tetracycline antibiotics and a mutation in at least one of the following nucleotide positions is detected with regard to reference genome NC_016822: 4595667, 4707429, 1655745, 4705326, 2876708, 1990704, 2539010, 1257335.

According to certain embodiments of the first and/or second aspect of the invention, the antibiotic drug is CF and a mutation in at least one of the following nucleotide positions is detected with regard to reference genome NC_016822: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the antibiotic drug is CFZ and a mutation in at least one of the following nucleotide positions is detected with regard to reference genome NC_016822: 3134050.

According to certain embodiments of the first and/or second aspect of the invention, the antibiotic drug is A/S and a mutation in at least one of the following nucleotide positions is detected with regard to reference genome NC_016822: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the antibiotic drug is CRM and a mutation in at least one of the following nucleotide positions is detected with regard to reference genome NC_016822: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the antibiotic drug is P/T and a mutation in at least one of the following nucleotide positions is detected with regard to reference genome NC_016822: 2241215, 4595667, 4502174, 2543074, 1159191, 2325584, 4707429, 3134050, 1655745, 949314, 1427818, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the antibiotic drug is AM and a mutation in at least one of the following nucleotide positions is detected with regard to reference genome NC_016822: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860.

According to certain embodiments of the first and/or second aspect of the invention, the antibiotic drug is AUG and a mutation in at least one of the following nucleotide positions is detected with regard to reference genome NC_016822: 2309519, 2314492, 4595667, 87512, 1159191, 2325584, 4707429, 3134050, 1427818, 2636621, 1429397, 2877526, 1200860.

According to certain embodiments of the first and/or second aspect of the invention, the antibiotic drug is TE and a mutation in at least one of the following nucleotide positions is detected with regard to reference genome NC_016822: 4595667, 4707429, 1655745, 4705326, 2876708, 1990704, 2539010, 1257335.

According to certain embodiments of the first and/or second aspect of the invention, the resistance of a bacterial microorganism belonging to the species Shigella against 1, 2, 3, 4, 5, 6, 7, 8, 9, 10, 11, 12, 13, 14, 15 or 16, 17, 18, 19, 20 or 21 antibiotic drugs is determined.

According to certain embodiments of the first and/or second aspect of the invention, a detected mutation is a mutation leading to an altered amino acid sequence in a polypeptide derived from a respective gene in which the detected mutation is located. According to this aspect, the detected mutation thus leads to a truncated or version of the polypeptide (wherein a new stop codon is created by the mutation) or a mutated version of the polypeptide having an amino acid exchange at the respective position.

According to certain embodiments of the first and/or second aspect of the invention, determining the nucleic acid sequence information or the presence of a mutation comprises determining a partial sequence or an entire sequence of the at least two genes.

According to certain embodiments of the first and/or second aspect of the invention, determining the nucleic acid sequence information or the presence of a mutation comprises determining a partial or entire sequence of the genome of the Shigella species, wherein said partial or entire sequence of the genome comprises at least a partial sequence of said at least two genes.

According to certain embodiments of the first and/or second aspect of the invention, determining the nucleic acid sequence information or the presence of a mutation comprises using a next generation sequencing or high throughput sequencing method. According to preferred embodiments of the first and/or second aspect of the invention, a partial or entire genome sequence of the bacterial organism of Shigella species is determined by using a next generation sequencing or high throughput sequencing method.

In a further, third aspect, the present invention relates to a method of determining an antimicrobial drug, e.g. antibiotic, resistance profile for bacterial microorganisms of Shigella species, comprising:
obtaining or providing a first data set of gene sequences of a plurality of clinical isolates of Shigella species;
providing a second data set of antimicrobial drug, e.g. antibiotic, resistance of the plurality of clinical isolates of Shigella species;
aligning the gene sequences of the first data set to at least one, preferably one, reference genome of Shigella, or assembling the gene sequence of the first data set, at least in part;
analyzing the gene sequences of the first data set for genetic variants to obtain a third data set of genetic variants;
correlating the third data set with the second data set and statistically analyzing the correlation; and
determining the genetic sites in the genome of Shigella associated with antimicrobial drug, e.g. antibiotic, resistance.

The different steps can be carried out as described with regard to the method of the first aspect of the present invention.

When referring to the second data set, wherein the second data set e.g. comprises, respectively is, a set of antimicrobial drug, e.g. antibiotic, resistances of a plurality of clinical isolates, this can, within the scope of the invention, also refer to a self-learning data base that, whenever a new sample is analyzed, can take this sample into the second data set and thus expand its data base. The second data set thus does not have to be static and can be expanded, either by external input or by incorporating new data due to self-learning. This is, however, not restricted to the third aspect of the invention, but applies to other aspects of the invention that refer to a second data set, which does not necessarily have to refer to antimicrobial drug resistance. The same applies, where applicable, to the first data set, e.g. in the third aspect.

According to certain embodiments, statistical analysis in the present methods is carried out using Fisher's test with p < 10⁻⁶, preferably p < 10⁻⁹, particularly p < 10⁻¹⁰.

The method of the third aspect of the present invention, as well as related methods, e.g. according to the 7^{th}, 10^{th}, 11^{th}, 14^{th} and 16^{th} aspect, can, according to certain embodiments, comprise correlating different genetic sites to each other. This way even higher statistical significance can be achieved.

According to certain embodiments of the method of the third aspect and related methods - as above, the second data set is provided by culturing the clinical isolates of Shigella species on agar plates provided with antimicrobial drugs, e.g. antibiotics, at different concentrations and the second data is obtained by taking the minimal concentration of the plates that inhibits growth of the respective Shigella species.

According to certain embodiments of the method of the third aspect and related methods, the antibiotic is at least one selected from the group of β-lactams, β-lactam inhibitors, quinolines and derivatives thereof, aminoglycosides, tetracyclines, and folate synthesis inhibitors, preferably Amoxicillin/K Clavulanate, Ampicillin, Aztreonam, Cefazolin, Cefepime, Cefotaxime, Ceftazidime, Ceftriaxone, Cefuroxime, Cephalothin, Ciprofloxacin, Ertapenem, Gentamicin, Imipenem, Levofloxacin, Meropenem, Piperacillin/Tazobactam, Ampicillin/Sulbactam, Tetracycline, Tobramycin, and Trimethoprim/Sulfamethoxazole.

According to certain embodiments of the method of the third aspect and related methods, the gene sequences in the third data set are comprised in at least one gene from the group of genes consisting of SSON53_12105, SSON53_12455, SSON53_12070, SSON53_12475, metH, SSON53_00425, SSON53_23830, SSON53_13575, flgE, SSON53 12500, SSON53_06660, SSON53_24790, SSON53_16770, SSON53_24480, SSON53_08770, SSON53_044795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, or from the genes listed in Table 5.

According to certain embodiments of the method of the third aspect and related methods, the genetic sites in the genome of Shigella associated with antimicrobial drug, e.g. antibiotic, resistance are at least comprised in one gene from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_3400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085.

According to certain embodiments of the method of the third aspect and related methods, the genetic variant has a point mutation, an insertion and or deletion of up to four bases, and/or a frameshift mutation, particularly a frameshift mutation in YP_005456953.1.

A fourth aspect of the present invention relates to a method of determining an antimicrobial drug, e.g. antibiotic, resistance profile for a bacterial microorganism belonging to the species Shigella comprising the steps of
a) obtaining or providing a sample containing or suspected of containing the bacterial microorganism;
b) determining the presence of a mutation in at least one gene of the bacterial microorganism as determined by the method of the third aspect of the invention;
wherein the presence of a mutation is indicative of a resistance to an antimicrobial drug, e.g. antibiotic, drug.

Steps a) and b) can herein be carried out as described with regard to the first aspect, as well as for the following aspects of the invention.

With this method, any mutations in the genome of Shigella species correlated with antimicrobial drug, e.g. antibiotic, resistance can be determined and a thorough antimicrobial drug, e.g. antibiotic, resistance profile can be established.

A fifth aspect of the present invention relates to a diagnostic method of determining an infection of a patient with Shigella species potentially resistant to antimicrobial drug treatment, which also can be described as method of determining an antimicrobial drug, e.g. antibiotic, resistant Shigella infection in a patient, comprising the steps of:
a) obtaining or providing a sample containing or suspected of containing a bacterial microorganism belonging to the species Shigella from the patient;
b) determining the presence of at least one mutation in at least one gene of the bacterial microorganism belonging to the species Shigella as determined by the method of the third aspect of the present invention, wherein the presence of said at least one mutation is indicative of an antimicrobial drug, e.g. antibiotic, resistant Shigella infection in said patient.

Again, steps a) and b) can herein be carried out as described with regard to the first aspect of the present invention.

According to this aspect, a Shigella infection in a patient can be determined using sequencing methods as well as a resistance to antimicrobial drugs, e.g. antibiotics, of the Shigella species be determined in a short amount of time compared to the conventional methods.

In a sixth aspect the present invention relates to a method of selecting a treatment of a patient suffering from an infection with a potentially resistant Shigella strain, e.g. an antimicrobial drug, e.g. antibiotic, resistant Shigella infection, comprising the steps of:
a) obtaining or providing a sample containing or suspected of containing a bacterial microorganism belonging to the species Shigella from the patient;
b) determining the presence of at least one mutation in at least one gene of the bacterial microorganism belonging to the species Shigella as determined by the method of the third aspect of the invention, wherein the presence of said at least one mutation is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

This method can be carried out similarly to the second aspect of the invention and enables a fast was to select a suitable treatment with antibiotics for any infection with an unknown Shigella species.

A seventh aspect of the present invention relates to a method of acquiring, respectively determining, an antimicrobial drug, e.g. antibiotic, resistance profile for a bacterial microorganisms of Shigella species, comprising:
obtaining or providing a first data set of gene sequences of a clinical isolate of Shigella species;
providing a second data set of antimicrobial drug, e.g. antibiotic, resistance of a plurality of clinical isolates of Shigella species;
aligning the gene sequences of the first data set to at least one, preferably one, reference genome of Shigella, or assembling the gene sequence of the first data set, at least in part;
analyzing the gene sequences of the first data set for genetic variants to obtain a third data set of genetic variants of the first data set;
correlating the third data set with the second data set and statistically analyzing the correlation; and
determining the genetic sites in the genome of Shigella of the first data set associated with antimicrobial drug, e.g. antibiotic, resistance.

With this method, antimicrobial drug, e.g. antibiotic, resistances in an unknown isolate of Shigella can be determined.

According to certain embodiments, the reference genome of Shigella is NC_016822 as annotated at the NCBI. According to certain embodiments, statistical analysis in the present methods is carried out using Fisher's test with p < 10⁻⁶, preferably p < 10⁻⁹, particularly p < 10⁻¹⁰. Also, according to certain embodiments, the method further comprises correlating different genetic sites to each other.

An eighth aspect of the present invention relates to a computer program product comprising computer executable instructions which, when executed, perform a method according to the fifth, sixth or seventh aspect of the present invention.

In certain embodiments the computer program product is one on which program commands or program codes of a computer program for executing said method are stored. According to certain embodiments the computer program product is a storage medium. The same applies to the computer program products of the aspects mentioned afterwards, i.e. the twelfth and fifteenth aspect of the present invention. As noted above, the computer program products of the present invention can be self-learning, e.g. with respect to the first and second data sets.

In order to obtain the best possible information from the highly complex genetic data and develop an optimum model for diagnostic and therapeutical uses as well as the methods of the present invention - which can be applied stably in clinical routine - a thorough in silico analysis can be necessary. The proposed principle is based on a combination of different approaches, e.g. alignment with at least one, preferably more reference genomes or assembly of the genome and correlation of mutations found in every sample, e.g. from each patient, with all references and drugs, e.g. antibiotics, and search for mutations which occur in several drug and several strains.

Using the above steps a list of mutations as well of genes is generated. These can be stored in databases and statistical models can be derived from the databases. The statistical models can be based on at least one or more mutations at least one or more genes. Statistical models that can be trained can be combined from mutations and genes. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, a genome or parts of the genome of a microorganism can be sequenced from a patient to be diagnosed. Afterwards, core characteristics can be derived from the sequence data which can be used to predict resistance. These are the points in the database used for the final model, i.e. at least one mutation or at least one gene, but also combinations of mutations, etc.

The corresponding characteristics can be used as input for the statistical model and thus enable a prognosis for new patients. Not only the information regarding all resistances of all microorganisms, e.g. of Shigella species, against all drugs, e.g. antibiotics, can be integrated in a computer decision support tool, but also corresponding directives (e.g. EUCAST) so that only treatment proposals are made that are in line with the directives.

A ninth aspect of the present invention relates to the use of the computer program product according to the eighth aspect for acquiring an antimicrobial drug, e.g. antibiotic, resistance profile for bacterial microorganisms of Shigella species or in a method of the third aspect of the invention.

According to a tenth aspect, the present invention relates to a method of acquiring, respectively determining, an antimicrobial drug, e.g. antibiotic, resistance profile for a microorganism, particularly a bacterial microorganism, comprising:
obtaining or providing a first data set of gene sequences of a clinical isolate of the microorganism;
providing a second data set of antimicrobial drug, e.g. antibiotic resistance, of a plurality of clinical isolates of the microorganism;
aligning the gene sequences of the first data set to at least one, preferably one, reference genome of the microorganism, or assembling the gene sequences of the first data set, at least in part;
analyzing the gene sequences of the first data set for genetic variants to obtain a third data set of genetic variants of the first data set;
correlating the third data set with the second data set of antimicrobial drug, e.g. antibiotic, resistance of a plurality of clinical isolates of the microorganism and statistically analyzing the correlation; and
determining the genetic sites in the genome of the clinical isolate of the microorganism of the first data set associated with antimicrobial drug, e.g. antibiotic, resistance.

Again, the steps can be carried out as described for corresponding steps in other aspects before. This method describes the general concept of acquiring an antimicrobial drug, e.g. antibiotic, resistance profile for any microorganism.

According to an eleventh aspect, a method of acquiring, respectively determining, an antimicrobial drug, e.g. antibiotic, resistance profile for at least one microorganism, particularly at least one bacterial microorganism, obtained from a patient, comprising: acquiring a first data set of gene sequences of the at least one microorganism;
providing at least one second data set of antimicrobial drug, e.g. antibiotic, resistance, of a plurality of clinical isolates of the microorganism;
aligning the gene sequences of the first data set to at least one, preferably one, reference genome of the microorganism, or assembling the gene sequence of the first data set, at least in part;
analyzing the gene sequences of the first data set for genetic variants to obtain a third data set of genetic variants of the first data set;
correlating the third data set with the at least one second data set of antimicrobial drug, e.g. antibiotic, resistance of a plurality of clinical isolates of the microorganism and statistically analyzing the correlation; and
determining the genetic sites in the genome of the at least one microorganism of the first data set associated with antimicrobial drug, e.g. antibiotic, resistance is described.

Again, the steps defined can be carried out as similar steps described in other methods before. This method discloses the general identification of antimicrobial drug, e.g. antibiotic, resistances in any sample, particularly by a general computer program product.

A simple read out concept for a diagnostic test as described in this aspect is shown schematically in Fig. 1.

According to Fig. 1, a sample 1, e.g. blood from a patient, is used for molecular testing 2, e.g. using next generation sequencing (NGS), and then a molecular fingerprint 3 is taken, e.g. in case of NGS a sequence of selected genomic/plasmid regions or the whole genome is assembled. This is then compared to a reference library 4, i.e. selected sequences or the whole sequence are/is compared to one or more reference sequences, and mutations (SNPs, sequence- gene additions/deletions, etc.) are correlated with susceptibility/ reference profile of reference strains in the reference library. The reference library 4 herein contains many genomes and is different from a reference genome. Then the result 5 is reported comprising ID (pathogen identification), i.e. a list of all (pathogenic) species identified in the sample, and AST (antimicrobial susceptibility testing), i.e. a list including a susceptibility /resistance profile for all species listed

According to certain embodiments, statistical analysis in the present method is carried out using Fisher's test with p < 10⁻⁶, preferably p < 10⁻⁹, particularly p < 10⁻¹⁰. Also, according to certain embodiments, the method further comprises correlating different genetic sites to each other.

A twelfth aspect of the present invention relates to a computer program product comprising computer executable instructions which, when executed, perform a method according to the 10^{th} or 11^{th} aspect of the present invention. These computer program products thus relate to general computer program products referring to any microorganisms, applying the principles which also apply for microorganisms of Shigella species as laid out before.

According to a thirteenth aspect the use of the computer program products of the thirteenth aspect in a method of the first, second, fourth, fifth or sixth aspect is disclosed.

In a fourteenth aspect a method of selecting a treatment of a patient having an infection with a microorganism, preferably a bacterial microorganism, particularly preferably a bacterial microorganisms of Shigella species, comprising: obtaining or providing a first data set comprising a gene sequence of at least one clinical isolate of the microorganism from the patient;
providing a second data set of antimicrobial drug, e.g. antibiotic, resistance of a plurality of clinical isolates of the microorganism;
aligning the gene sequences of the first data set to at least one, preferably one, reference genome of the microorganism, or assembling the gene sequence of the first data set, at least in part;
analyzing the gene sequences of the first data set for genetic variants to obtain a third data set of genetic variants of the first data set;
correlating the third data set with the second data set of antimicrobial drug, e.g. antibiotic, resistance of a plurality of clinical isolates of the microorganism and statistically analyzing the correlation;
determining the genetic sites in the genome of the clinical isolate of the microorganism of the first data set associated with antimicrobial drug, e.g. antibiotic, resistance; and selecting a treatment of the patient with one or more antimicrobial, e.g. antibiotic, drugs different from the ones identified in the determination of the genetic sites associated with antimicrobial drug, e.g. antibiotic, resistance is disclosed.

Again, the steps can be carried out as similar steps before. In this method, as well as similar ones, no aligning is necessary, as the unknown sample can be directly correlated, after the genome or genome sequences are produced, with the second data set and thus mutations and antimicrobial drug, e.g. antibiotic, resistances can be determined. The first data set can be assembled, for example, using known techniques.

According to certain embodiments, statistical analysis in the present method is carried out using Fisher's test with p < 10⁻⁶, preferably p < 10⁻⁹, particularly p < 10⁻¹⁰. Also, according to certain embodiments, the method further comprises correlating different genetic sites to each other.

A fifteenth aspect of the present invention is directed to a computer program product comprising computer executable instructions which, when executed, perform a method according to the fourteenth aspect.

In a sixteenth aspect the present invention relates to a method of finding a pharmaceutical target for treating an infection with at least one microorganism, particularly at least one bacterial microorganism, comprising:
obtaining or providing a first data set of gene sequences of a plurality of clinical isolates of the at least one microorganism;
providing a second data set of sequences of the at least one microorganism representing genes relevant to a pharmaceutical compound;
aligning the gene sequences of the first data set to at least one, preferably one, reference genome each of the at least one microorganism, or assembling the gene sequence of the first data set, at least in part;
analyzing the gene sequences of the first data set for genetic variants to obtain a third data set of genetic variants of the first data set;
correlating the third data set to the second data set of sequences of the at least one microorganism representing genes relevant to a pharmaceutical compound; and determining the genetic sites in the genome of the clinical isolate of the microorganism of the first data set associated relevant to a pharmaceutical compound.

According to certain embodiments the sequences of the microorganism representing genes relevant to a pharmaceutical compound represent binding sites of the microorganism. These are likely to be targets for drugs, e.g. antibiotics.

Again, the principle of the method can be conducted using a computer program product.

Algorithms that can be used in such a computer program product showed for cases of single base exchanges (e.g. gyrase) as well as deletion of whole genes or open reading frames (e.g. beta lactamase) that known targets for drugs applied successfully on the marked can be found. It is to be expected that the data is perfectly suitable for finding further drug targets / pharmaceutical targets (many different organisms that are not monoclonal but very diverse in temporal and geographical aspects). For this purpose it can be tested for all highly significant mutations if the mutation is in a gene relevant for drugs, e.g. the binding site of a medicine). The results are of diagnostic interest. The findings can be prioritized. For example, for single base exchanges preferably results will be considered which are in binding sites. The respective binding sites most likely have a function in the metabolism of the drug, so that a mutation will cause an increased or decreased or lost effectiveness. Corresponding genes or even binding partners of the genes present presumed new targets for drugs which can enhance effects of existing therapies or work as single therapies.

In order to limit the number of candidates, all proteins with unknown function or hypothetical proteins can be excluded, concentrating on genes that are found in several organisms, e.g. more than ten, independently of one another.

According to a seventeenth aspect of the present invention, a diagnostic method of determining an infection of a patient with Shigella species potentially resistant to antimicrobial drug treatment, which can also be described as a method of determining an antimicrobial drug, e.g. antibiotic, resistant Shigella infection of a patient is disclosed, comprising the steps of:
a) obtaining or providing a sample containing or suspected of containing at least one Shigella species from the patient;
b) determining the presence of at least one mutation in at least two genes from the group of genes listed in Table 5, wherein the presence of said at least two mutations is indicative of an antimicrobial drug, e.g. antibiotic, resistant Shigella infection in said patient.

An eighteenth aspect of the invention discloses a method of selecting a treatment of a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least two genes from the group of genes listed in Table 5, wherein the presence of said at least two mutations is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

Again, the steps can be carried out as in similar methods before, e.g. as in the first and second aspect of the invention. In the seventeenth and eighteenth aspect of the invention, all classes of antibiotics considered in the present method are covered.

Herein, the genes in Table 5 are the following:
SSON53_16770, SSON53_19730, SSON53_14680, SSON53_09620, SSON53_25600, metH, SSON53_24790, SSON53_13530, SSON53_12100, SSON53_12655, SSON53_04070, SSON53_21285, SSON53_01970, held, SSON53_08660, SSON53_14430, SSON53_17515, SSON53_11020, SSON53_00015, SSON53_01080, SSON53_12830, SSON53_13420, SSON53_13780, SSON53_16000, SSON53_16880, SSON53_18540, SSON53 22785, SSON53_24145, SSON53_25200, SSON53_25460, SSON53_03770, SSON53_18890, SSON53_16535, SSON53_19285, SSON53_03650, SSON53_15285, SSON53_18775, SSON53_17120, SSON53_24410, SSON53_26285, tig, SSON53_07220, SSON53_08070, SSON53_17780, gcvT, SSON53_20935, pyrB, SSON53_12595, SSON53_04605, SSON53_07910, SSON53_13390, queF, SSON53_18505, pdxA, SSON53_00645, SSON53_01515, SSON53_02505, SSON53_03060, dpiB, SSON53_03230, SSON53_03255, SSON53_04495, SSON53_05165, SSON53_05595, SSON53_06535, SSON53_06585, SSON53_07385, SSON53_07390, SSON53_08640, SSON53_08945, SSON53_10840, SSON53_12340, pbpG, SSON53_13270, SSON53_14190, SSON53_14265, SSON53_14460, murP, SSON53_14705, SSON53_14835, SSON53_15125, SSON53_16590, SSON53_16955, rumA, recD, SSON53_17670, SSON53_17690, SSON53_17705, SSON53_17955, SSON53_18080, SSON53 18610, SSON53 18735, SSON53 18885, SSON53 00360, thiP, SSON53 00595, SSON53 02980, SSON53 03520, SSON53 03560, bioD, SSON53_04325, SSON53_04710, putA, SSON53_05585, rne, SSON53_07205, SSON53_08060, SSON53_08955, SSON53_08960, SSON53_09205, SSON53_10160, SSON53_11100, dacD, SSON53_13065, fadJ, SSON53_14085, SSON53_14455, murQ, SSON53_16065, SSON53_16755, SSON53_18520, SSON53_20320, SSON53_21970, fadB, SSON53_24280, SSON53_25150, SSON53_25295, SSON53_25400, SSON53_00025, SSON53_04285, SSON53_04300, SSON53_06415, SSON53_07990, SSON53_08770, SSON53_09680, SSON53_10655, SSON53_11810, SSON53_12330, SSON53_12740, SSON53_12795, SSON53_13290, SSON53_13415, SSON53_13555, SSON53_15280, SSON53_16180, SSON53_19150, SSON53_22470, SSON53_24775, SSON53_24780, SSON53_02695, SSON53_04540, wcaD, SSON53_14020, SSON53_14025, SSON53_21860, and SSON53_22935.

According to certain embodiments, mutations in at least two, three, four, five, six, seven, eight, nine or ten genes are determined in any of the methods of the present invention, e.g. in at least two genes or in at least three genes. Instead of testing only single genes or mutants, a combination of several variant positions can improve the prediction accuracy and further reduce false positive findings that are influenced by other factors. Therefore, it is in particular preferred to determine the presence of a mutation in 2, 3, 4, 5, 6, 7, 8 or 9 (or more) genes selected from Table 5.

**Table 5: List of genes**

| | | | | | |
|---|---|---|---|---|---|
| SSON53_16770 | SSON53_319730 | SSON53_14680 | SSON53_09620 | SSON53_25600 | metH |
| SSON53_24790 | SSON53_13530 | SSON53_12100 | SSON53_12655 | SSON53_04070 | SSON53_21285 |
| SSON53_01970 | helD | SSON53_08660 | SSON53_14430 | SSON53_17515 | SSON53_11020 |
| SSON53_00015 | SSON53_01080 | SSON53_12830 | SSON53_13420 | SSON53_13780 | SSON53_16000 |
| SSON53_16880 | SSON53_18540 | SSON53_22785 | SSON53_24145 | SSON53_25200 | SSON53_25460 |
| SSON53_03770 | SSON53_18890 | SSON53_16535 | SSON53_19285 | SSON53_03650 | SSON53_15285 |
| SSON53_18775 | SSON53_17120 | SSON53_24410 | SSON53_26285 | tig | SSON53_07220 |
| SSON53_08070 | SSON53_17780 | gcvT | SSON53_20935 | pyrB | SSON53_12595 |
| SSON53_04605 | SSON53_07910 | SSON53_13390 | queF | SSON53_18505 | pdxA |
| SSON53_00645 | SSON53_01515 | SSON53_02505 | SSON53_03060 | dpiB | SSON53_03230 |
| SSON53_03255 | SSON53_04495 | SSON53_05165 | SSON53_05595 | SSON53_06535 | SSON53_06585 |
| SSON53_07385 | SSON53_07390 | SSON53_08640 | SSON53_08945 | SSON53_10840 | SSON53_12340 |
| pbpG | SSON53_13270 | SSON53_14190 | SSON53_14265 | SSON53_14460 | murP |
| SSON53_14705 | SSON53_14835 | SSON53_15125 | SSON53_16590 | SSON53_16955 | rumA |
| recD | SSON53_17670 | SSON53_17690 | SSON53_17705 | SSON53_17955 | SSON53_18080 |
| SSON53_18610 | SSON53_18735 | SSON53_18885 | SSON53_00360 | thiP | SSON53_00595 |
| SSON53_02980 | SSON53_03520 | SSON53_03560 | bioD | SSON53_04325 | SSON53_04710 |
| putA | SSON53_05585 | rne | SSON53_07205 | SSON53_08060 | SSON53_08955 |
| SSON53_08960 | SSON53_09205 | SSON53_10160 | SSON53_11100 | dacD | SSON53_13065 |
| fadJ | SSON53_14085 | SSON53_14455 | murQ | SSON53_16065 | SSON53_16755 |
| SSON53_18520 | SSON53_20320 | SSON53_21970 | fadB | SSON53_24280 | SSON53_25150 |
| SSON53_25295 | SSON53_25400 | SSON53_00025 | SSON53_04285 | SSON53_04300 | SSON53_06415 |
| SSON53_07990 | SSON53_08770 | SSON53_09680 | SSON53_10655 | SSON53_11810 | SSON53_12330 |
| SSON53_12740 | SSON53_12795 | SSON53_13290 | SSON53_13415 | SSON53_13555 | SSON53_15280 |
| SSON53_16180 | SSON53_19150 | SSON53_22470 | SSON53_24775 | SSON53_24780 | SSON53_02695 |
| SSON53_04540 | wcaD | SSON53_14020 | SSON53_14025 | SSON53_21860 | SSON53_22935 |

Further, according to certain embodiments, the reference genome of Shigella is again NC_016822 as annotated at the NCBI. According to certain embodiments, statistical analysis in the present methods is carried out using Fisher's test with p < 10⁻⁶, preferably p < 10⁻⁹, particularly p < 10⁻¹⁰. Also, according to certain embodiments, the method further comprises correlating different genetic sites to each other. Also the other aspects of the embodiments of the first and second aspect of the invention apply.

According to certain embodiments of the method of the seventeenth and/or eighteenth aspect of the present invention, the antimicrobial drug is an antibiotic. According to certain embodiments, the antibiotic is a lactam antibiotic and a mutation in at least one of the genes listed in Table 6 or 7 is detected, or a mutation in at least one of the positions (denoted POS in the tables) listed in Table 6 or 7.

**Table 6: List for lactam antibiotics (all 7 antibiotics)**

| **gene name** | **POS** | **antibiotic** | **p-value (FDR)** | **genbank protein accession number** |
|---|---|---|---|---|
| SSON53_16770 | 3134050 | CF;CFZ;CRM;P/T;AM;A/S;AUG | 4,9931E-41 | YP_005457860.1 |
| SSON53_19730 | 3722816 | CF;CFZ;CRM;P/T;AM;A/S;AUG | 8,7799E-37 | YP_005458448.1 |
| SSON53_14680 | 2748494 | CF;CFZ;CRM;P/T;AM;A/S;AUG | 1,9267E-34 | YP_005457450.1 |
| SSON53_09620 | 1816305 | CF;CFZ;CRM;P/T;AM;A/S;AUG | 2,5314E-34 | YP_005456470.1 |
| SSON53_25600 | 4860933 | CF;CFZ;CRM;P/T;AM;A/S;AUG | 2,636E-33 | YP_005459592.1 |

| | | | | |
|---|---|---|---|---|
| FDR: determined according to FDR (Benjamini Hochberg) method (Benjamini Hochberg, 1995) | | | | |

According to certain embodiments of the method of the seventeenth and/or eighteenth aspect of the present invention, the antibiotic is at least one of CF, CFZ, CRM, P/T, AM, A/S, and AUG and a mutation in at least one of the genes of SSON53_16770, SSON53_19730, SSON53_14680, SSON53_09620, SSON53_25600 is detected, or a mutation in at least one of the positions of 3134050, 3722816, 2748494, 1816305, 4860933.

**Table 7: List for lactam antibiotics (6 antibiotics)**

| **gene name** | **POS** | **antibiotic** | **p-value (FDR)** | **genbank protein accession number** |
|---|---|---|---|---|
| metH | 4595667 | CF;TE;A/S;CRM;P/T;AM;AUG | 9,1881E-42 | YP_005459323.1 |
| SSON53_24790 | 4707429 | CF;TE;A/S;CRM;P/T;AM;AUG | 4,3278E-41 | YP_005459432.1 |
| SSON53_13530 | 2533894 | CF;TE;A/S;CRM;P/T;AM;AUG | 8,4286E-40 | YP_005457233.1 |
| SSON53_12100 | 2239494 | CF;TE;A/S;CRM;P/T;AM;AUG | 1,7469E-38 | YP_005456952.1 |
| SSON53_12655 | 2352355 | CF;TE;A/S;CRM;P/T;AM;AUG | 3,1716E-38 | YP_005457061.1 |
| SSON53_04070 | 802768 | CF;TE;A/S;CRM;P/T;AM;AUG | 4,3241E-32 | YP_005455388.1 |
| SSON53_21285 | 4005875 | CF;TE;A/S;CRM;P/T;AM;AUG | 2,6354E-27 | YP_005458753.1 |
| SSON53_01970 | 399928 | CF;TE;CFZ;CRM;AM;A/S;AUG | 1,7083E-26 | YP_005454973.1 |
| helD | 1045235 | CF;TE;CFZ;CRM;AM;A/S;AUG | 1,7083E-26 | YP_005455617.1 |
| SSON53_08660 | 1633619 | CF;TE;CFZ;CRM;AM;A/S;AUG | 1,7083E-26 | YP_005456280.1 |
| SSON53_14430 | 2697830 | CF;TE;CFZ;CRM;AM;A/S;AUG | 1,7083E-26 | YP_005457402.1 |
| SSON53_17515 | 3283400 | CF;TE;CFZ;CRM;AM;A/S;AUG | 1,7083E-26 | YP_005458007.1 |
| SSON53_11020 | 2053683 | CF;TE;CFZ;CRM;AM;A/S;AUG | 2,0794E-26 | YP_005456742.1 |
| SSON53_00015 | 3915 | CF;TE;CFZ;CRM;AM;A/S;AUG | 2,2002E-26 | YP_005454592.1 |
| SSON53_01080 | 226692 | CF;TE;CFZ;CRM;AM;A/S;AUG | 2,2002E-26 | YP_005454803.1 |

According to certain embodiments of the method of the seventeenth and/or eighteenth aspect of the present invention, the antibiotic is at least one of CF, A/S, CRM, AM, and AUG and a mutation in at least one of the genes of metH, SSON53_24790, SSON53_13530, SSON53_12100, SSON53_12655, SSON53_04070, SSON53_21285, SSON53_01970, held, SSON53_08660, SSON53_14430, SSON53_17515, SSON53_11020, SSON53_00015, SSON53_01080 is detected, or a mutation in at least one of the positions of 4595667, 4707429, 2533894, 2239494, 2352355, 802768, 4005875, 399928, 1045235, 1633619, 2697830, 3283400, 2053683, 3915, 226692.

According to certain embodiments of the method of the seventeenth and/or eighteenth aspect of the present invention, the antibiotic is P/T_and a mutation in at least one of the genes of metH, SSON53_24790, SSON53_13530, SSON53_12100, SSON53_12655, SSON53_04070, SSON53_21285 is detected, or a mutation in at least one of the positions of 4595667, 4707429, 2533894, 2239494, 2352355, 802768, 4005875.

According to certain embodiments of the method of the seventeenth and/or eighteenth aspect of the present invention, the antibiotic is CFZ and a mutation in at least one of the genes of SSON53_01970, held, SSON53_08660, SSON53_14430, SSON53_17515, SSON53_11020, SSON53_00015, SSON53_01080 is detected, or a mutation in at least one of the positions of 399928, 1045235, 1633619, 2697830, 3283400, 2053683, 3915, 226692.

According to certain embodiments of the method of the seventeenth and/or eighteenth aspect of the present invention, the antibiotic is a quinolone antibiotic and a mutation in at least one of the genes listed in Table 8 or 9 is detected, or a mutation in at least one of the positions (denoted POS in the tables) listed in Table 8 or 9.

**Table 8: List for quinolone antibiotics (all 2)**

| **gene name** | **POS** | **drug** | **p-value (FDR)** | **genbank protein accession number** |
|---|---|---|---|---|
| SSON53_04605 | 905340 | CP;LVX | 4,0068E-10 | YP_005455495.1 |
| SSON53_07910 | 1506051 | CP;LVX | 4,2234E-10 | YP_005456134.1 |
| SSON53_ 13390 | 2503366 | CP;LVX | 3,9244E-10 | YP_005457205.1 |
| queF | 3228225 | CP;LVX | 3,9244E-10 | YP_005457963.1 |
| SSON53_ 18505 | 3476068 | CP;LVX | 3,9244E-10 | YP_005458205.1 |

According to certain embodiments of the method of the seventeenth and/or eighteenth aspect of the present invention, the antibiotic is at least one of CP and LVX and a mutation in at least one of the genes of SSON53_04605, SSON53_07910, SSON53_13390, queF, SSON53_18505 is detected, or a mutation in at least one of the positions of 905340, 1506051, 2503366, 3228225, 3476068.

**Table 9: List for quinolone antibiotics (1 antibiotic)**

| **gene name** | **POS** | **drug** | **p-value (FDR)** | **genbank protein accession number** |
|---|---|---|---|---|
| SSON53_13390 | 2503749 | CP | 3,4593E-31 | YP_005457205.1 |
| SSON53_19730 | 3723920 | CP | 2,2396E-13 | YP_005458448.1 |
| SSON53_119730 | 3723919 | CP | 8,7463E-13 | YP_005458448.1 |
| SSON53_19730 | 3723923 | CP | 8,7463E-13 | YP_005458448.1 |
| SSON53_19730 | 3723925 | CP | 1,2893E-12 | YP_005458448.1 |
| SSON53_16955 | 3164176 | CP | 2,7682E-12 | YP_005457897.1 |
| SSON53_05595 | 1110022 | CP | 3,7569E-12 | YP_005455683.1 |
| SSON53_07390 | 1422899 | CP | 4,7319E-12 | YP_005456032.1 |
| SSON53_15125 | 2846241 | CP | 4,8574E-12 | YP_005457535.1 |
| dpiB | 619830 | T/S;CP | 8,2584E-12 | YP_005455196.1 |
| SSON53_07385 | 1421710 | CP | 9,2316E-12 | YP_005456031.1 |
| SSON53_17670 | 3315886 | CP | 1,7472E-11 | YP_005458038.1 |
| SSON53_14460 | 2704526 | T/S;CP | 3,1286E-11 | YP_005457408.1 |
| SSON53_19730 | 3722696 | CP | 3,6609E-11 | YP_005458448.1 |
| SSON53_21860 | 4105284 | T/S;CP | 4,6957E-11 | YP_005458864.1 |

According to certain embodiments of the method of the seventeenth and/or eighteenth aspect of the present invention, the antibiotic is CP and a mutation in at least one of the genes of SSON53_13390, SSON53_19730, SSON53_16955, SSON53_05595, SSON53_07390, SSON53_15125, dpiB, SSON53_07385, SSON53_17670, SSON53_14460, SSON53_21860 is detected, or a mutation in at least one of the positions of 2503749, 3723920, 3723919, 3723923, 3723925, 3164176, 1110022, 1422899, 2846241, 619830, 1421710, 3315886, 2704526, 3722696, 4105284.

According to certain embodiments of the method of the seventeenth and/or eighteenth aspect of the present invention, the antibiotic is an aminoglycoside antibiotic and a mutation in at least one of the genes listed in Table 10 is detected, or a mutation in at least one of the positions (denoted POS in the tables) listed in Table 10.

**Table 10: List of aminoglycoside antibiotics**

| **gene name** | **POS** | **drug** | **p-value (FDR)** | **genbank protein accession number** |
|---|---|---|---|---|
| SSON53_04325 | 847461 | TO | 5,1737E-12 | YP_005455439.1 |
| SSON53_04710 | 927380 | TO | 5,1737E-12 | YP_005455516.1 |
| SSON53_14455 | 2702371 | TO | 5,1737E-12 | YP_005457407.1 |
| SSON53_16755 | 3130278 | TO | 5,1737E-12 | YP_005457857.1 |
| SSON53_03520 | 707965 | TO | 5,294E-12 | YP_005455281.1 |
| SSON53_16065 | 3013091 | TO | 5,294E-12 | YP_005457721.1 |
| SSON53_00360 | 74735 | TO | 1,7111E-11 | YP_005454661.1 |
| SSON53_00595 | 124368 | TO | 1,7111E-11 | YP_005454708.1 |
| SSON53_25150 | 4777150 | TO | 1,7111E-11 | YP_005459502.1 |
| fadB | 4366586 | TO | 1,7908E-11 | YP_005459106.1 |
| SSON53_10160 | 1917725 | TO | 1,9527E-11 | YP_005456572.1 |
| SSON53_02980 | 600143 | TO | 2,5118E-11 | YP_005455173.1 |
| SSON53_02980 | 600209 | TO | 2,5118E-11 | YP_005455173.1 |
| SSON53_08960 | 1693067 | TO | 3,1643E-11 | YP_005456340.1 |
| SSON53_03560 | 714500 | TO | 5,1292E-11 | YP_005455289.1 |

According to certain embodiments of the method of the seventeenth and/or eighteenth aspect of the present invention, the antibiotic is TO and a mutation in at least one of the genes of SSON53_04325, SSON53_04710, SSON53_14455, SSON53_16755, SSON53_03520, SSON53_16065, SSON53_00360, SSON53_00595, SSON53_25150, fadB, SSON53_10160, SSON53_02980, SSON53_02980, SSON53_08960, SSON53_03560 is detected, or a mutation in at least one of the positions of 847461, 927380, 2702371, 3130278, 707965, 3013091, 74735, 124368, 4777150, 4366586, 1917725, 600143, 600209, 1693067, 714500.

According to certain embodiments of the method of the seventeenth and/or eighteenth aspect of the present invention, the antibiotic is an polyketide antibiotic and a mutation in at least one of the genes listed in Table 11 is detected, or a mutation in at least one of the positions (denoted POS in the tables) listed in Table 11.

**Table 11: List of polyketides, preferably tetracycline**

| **gene name** | **POS** | **Drug** | **p-value (FDR)** | **genbank protein accession number** |
|---|---|---|---|---|
| metH | 4595667 | CF;TE;A/S;CRM;P/T;AM;AUG | 9,1881E-42 | YP_005459323.1 |
| SSON53_24790 | 4707429 | CF;TE;A/S;CRM;P/T;AM;AUG | 4,3278E-41 | YP_005459432.1 |
| SSON53_08770 | 1655745 | CF;TE;A/S;CRM;P/T;AM | 6,6062E-41 | YP_005456302.1 |
| SSON53_08770 | 1655894 | CF;TE;A/S;CRM;P/T;AM | 6,6062E-41 | YP_005456302.1 |
| SSON53_24780 | 4705326 | CF;TE;A/S;CRM;P/T;AM | 1,2525E-40 | YP_005459430.1 |
| SSON53_15280 | 2876708 | CF;TE;A/S;CRM;P/T;AM | 1,7165E-40 | YP_005457566.1 |
| SSON53_10655 | 1990704 | CF;TE;A/S;CRM;P/T;AM | 3,7364E-40 | YP_005456671.1 |
| SSON53_13555 | 2539010 | CF;TE;A/S;CRM;P/T;AM | 3,7364E-40 | YP_005457238.1 |
| SSON53_06415 | 1257335 | CF;TE;A/S;CRM;P/T;AM | 3,8276E-40 | YP_005455839.1 |
| SSON53_04285 | 840523 | CF;TE;A/S;CRM;P/T;AM | 8,4286E-40 | YP_005455431.1 |
| SSON53_04300 | 842535 | CF;TE;A/S;CRM;P/T;AM | 8,4286E-40 | YP_005455434.1 |
| SSON53_13530 | 2533894 | CF;TE;A/S;CRM;P/T;AM;AUG | 8,4286E-40 | YP_005457233.1 |
| SSON53_13555 | 2539007 | CF;TE;A/S;CRM;P/T;AM | 8,4286E-40 | YP_005457238.1 |
| SSON53_19150 | 3604284 | CF;TE;A/S;CRM;P/T;AM | 8,4286E-40 | YP_005458332.1 |
| SSON53_24775 | 4703410 | CF;TE;A/S;CRM;P/T;AM | 3,6652E-39 | YP_005459429.1 |
| SSON53_12100 | 2239494 | CF;TE;A/S;CRM;P/T;AM;AUG | 1,7469E-38 | YP_005456952.1 |
| SSON53_12795 | 2378416 | CF;TE;A/S;CRM;P/T;AM | 1,8821E-38 | YP_005457089.1 |

According to certain embodiments of the method of the seventeenth and/or eighteenth aspect of the present invention, the antibiotic is TE and a mutation in at least one of the genes of metH, SSON53_24790, SSON53_08770, SSON53_24780, SSON53_15280, SSON53_10655, SSON53_13555, SSON53_06415, SSON53_04285, SSON53_04300, SSON53 13530, SSON53_19150, SSON53_24775, SSON53_12100, SSON53_12795 is detected, or a mutation in at least one of the positions of 4595667, 4707429, 1655745, 1655894, 4705326, 2876708, 1990704, 2539010, 1257335, 840523, 842535, 2533894, 2539007, 3604284, 4703410, 2239494, 2378416.

According to certain embodiments of the method of the seventeenth and/or eighteenth aspect of the present invention, the antibiotic is T/S and a mutation in at least one of the genes listed in Table 12 is detected, or a mutation in at least one of the positions (denoted POS in the tables) listed in Table 12.

**Table 12: List of others antibiotics**

| **gene name** | **POS** | **drug** | **p-value (FDR)** | **genbank protein accession number** |
|---|---|---|---|---|
| SSON53_14020; S N53_14025(*) | 2627369 | T/S;A/S;TE;AM | 7,9267E-18 | YP_005457324.1;YP _005457325.1 |
| SSON53_04540 | 894506 | T/S;TE | 4,455E-15 | YP_005455482.1 |
| dpiB | 619830 | T/S;CP | 8,2584E-12 | YP_005455196.1 |
| SSON53_14460 | 2704526 | T/S;CP | 3,1286E-11 | YP_005457408.1 |
| SSON53_21860 | 4105284 | T/S;CP | 4,6957E-11 | YP_005458864.1 |
| SSON53_22935 | 4337358 | T/S;CP;AM | 5,9394E-11 | YP_005459075.1 |
| SSON53_17690 | 3320136 | T/S;CP | 1,8593E-10 | YP_005458042.1 |
| wcaD | 2292673 | T/S;TE | 1,9564E-10 | YP_005457009.1 |
| SSON53_17705 | 3327522 | T/S;CP | 2,0575E-10 | YP_005458045.1 |
| SSON53_02695 | 538074 | T/S | 2,62E-10 | YP_005455116.1 |

| | | | | |
|---|---|---|---|---|
| (*) the single nucleotide polymorphism in this case is in both genes | | | | |

A nineteenth aspect of the present invention is directed to a diagnostic method of determining an infection of a patient with Shigella species potentially resistant to antimicrobial drug treatment, which can also be described as method of determining an antimicrobial drug, e.g. antibiotic, resistant Shigella infection of a patient, comprising the steps of:
a) obtaining or providing a sample containing or suspected of containing at least one Shigella species from the patient;
b) determining the presence of at least one mutation in at least one gene from the group of genes consisting of SSON53_12105, SSON53_12455, SSON53_12070, SSON53_12475, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, and pdxA, or from the group of genes consisting of SSON53_12105, SSON53_12455, SSON53_12475, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, pdxA, SSON53_04690, SSON53_04945, SSON53_06005, and SSON53_06085, wherein the presence of said at least one mutation is indicative of an antimicrobial drug, e.g. antibiotic, resistant Shigella infection in said patient.

A twentieth aspect of the present invention is directed to a method of selecting a treatment of a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least one gene from the group of genes consisting of SSON53_12105, SSON53_12455, SSON53_12070, SSON53_12475, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, and pdxA, or from the group of genes consisting of SSON53_12105, SSON53_12455, SSON53_12475, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, pdxA, SSON53_04690, SSON53_04945, SSON53_06005, and SSON53_06085, wherein the presence of said at least one mutation is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

Again, in the nineteenth and the twentieth aspect the steps correspond to those in the first or second aspect, although only a mutation in at least one gene is determined.

A twenty-first aspect of the present invention is directed to a method of treating a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least one gene from the group of genes consisting of SSON53_12105, SSON53_12455, SSON53_12070, SSON53_12475, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, and pdxA, or from the group of genes consisting of SSON53_12105, SSON53_12455, SSON53_12475, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, SSON53_02755, SSON53_06415, SSON53_04615, SSON53_17330, pdxA, SSON53_04690, SSON53_04945, SSON53_06005, and SSON53_06085, wherein the presence of said at least one mutation is indicative of a resistance to one or more antimicrobial, e.g. antibiotic, drugs;
c) identifying said at least one or more antimicrobial, e.g. antibiotic, drugs;
d) selecting one or more antimicrobial, e.g. antibiotic, drugs different from the ones identified in step c) and being suitable for the treatment of a Shigella infection; and
e) treating the patient with said one or more antimicrobial, e.g. antibiotic, drugs.

A twenty-second aspect of the present invention is directed to method of treating a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of a resistance to one or more antimicrobial, e.g. antibiotic, drugs;
c) identifying said at least one or more antimicrobial, e.g. antibiotic, drugs;
d) selecting one or more antimicrobial, e.g. antibiotic, drugs different from the ones identified in step c) and being suitable for the treatment of a Shigella infection; and
e) treating the patient with said one or more antimicrobial, e.g. antibiotic, drugs.

A twenty-third aspect of the present invention is directed to method of treating a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least two genes from the group of genes listed in Table 5, wherein the presence of said at least two mutations is indicative of a resistance to one or more antimicrobial, e.g. antibiotic, drugs;
c) identifying said at least one or more antimicrobial, e.g. antibiotic, drugs;
d) selecting one or more antimicrobial, e.g. antibiotic, drugs different from the ones identified in step c) and being suitable for the treatment of a Shigella infection; and
e) treating the patient with said one or more antimicrobial, e.g. antibiotic, drugs.

A twenty-fourth aspect of the present invention is directed to method of treating a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least one gene from the group of genes listed in Table 13, wherein the presence of said at least one mutation is indicative of a resistance to one or more antimicrobial, e.g. antibiotic, drugs;
c) identifying said at least one or more antimicrobial, e.g. antibiotic, drugs;
d) selecting one or more antimicrobial, e.g. antibiotic, drugs different from the ones identified in step c) and being suitable for the treatment of a Shigella infection; and
e) treating the patient with said one or more antimicrobial, e.g. antibiotic, drugs.

Also in the twenty-first to twenty-fourth aspect of the invention, steps a) to d) are analogous to the steps in the method of the second aspect of the present invention. Step e) can be sufficiently carried out without being restricted and can be done e.g. non-invasively.

A twenty-fifth aspect of the present invention is directed to a diagnostic method of determining an infection of a patient with Shigella species potentially resistant to antimicrobial drug treatment, which can also be described as method of determining an antimicrobial drug, e.g. antibiotic, resistant Shigella infection of a patient, comprising the steps of:
a) obtaining or providing a sample containing or suspected of containing at least one Shigella species from the patient;
b) determining the presence of at least one mutation in at least one gene from the group of genes listed in Table 13, preferably from the group of genes listed in Table 14, wherein the presence of said at least one mutation is indicative of an antimicrobial drug, e.g. antibiotic, resistant Shigella infection in said patient.

A twenty-sixth of the present invention is directed to a method of selecting a treatment of a patient suffering from an antimicrobial drug, e.g. antibiotic, resistant Shigella infection, comprising the steps of:
a) obtaining or providing a sample containing or suspected of containing at least one Shigella species from the patient;
b) determining the presence of at least one mutation in at least one gene from the group of genes listed in Table 13, preferably from the group of genes listed in Table 14, wherein the presence of said at least one mutation is indicative of a resistance to one or more antimicrobial, e.g. antibiotic, drugs;
c) identifying said at least one or more antimicrobial, e.g. antibiotic, drugs; and
d) selecting one or more antimicrobial, e.g. antibiotic, drugs different from the ones identified in step c) and being suitable for the treatment of a Shigella infection.

Again, in the twenty-fifth and the twenty-sixth aspect the steps correspond to those in the first or second aspect, although only a mutation in at least one gene is determined.

**Table 13: List of genes**

| | | | | | |
|---|---|---|---|---|---|
| SSON53_16770 | SSON53_19730 | SSON53_14680 | SSON53_09620 | SSON53_25600 | SSON53_22935 |
| SSON53_24790 | SSON53_13530 | SSON53_12100 | SSON53_12655 | SSON53_04070 | SSON53_21285 |
| SSON53_01970 | wcaD | SSON53_08660 | SSON53_14430 | SSON53_17515 | SSON53_11020 |
| SSON53_00015 | SSON53_01080 | SSON53_12830 | SSON53_13420 | SSON53_13780 | SSON53_16000 |
| SSON53_16880 | SSON53_18540 | SSON53_22785 | SSON53_24145 | SSON53_25200 | SSON53_25460 |
| SSON53_03770 | SSON53_18890 | SSON53_16535 | SSON53_19285 | SSON53_03650 | SSON53_15285 |
| SSON53_18775 | SSON53_17120 | SSON53_24410 | SSON53_26285 | SSON53_14025 | SSON53_07220 |
| SSON53_08070 | SSON53_17780 | gcvT | SSON53_20935 | SSON53_21860 | SSON53_12595 |
| SSON53_04605 | SSON53_07910 | SSON53_13390 | queF | SSON53_18505 | pdxA |
| SSON53_00645 | SSON53_01515 | SSON53_02505 | SSON53_03060 | dpiB | SSON53_03230 |
| SSON53_03255 | SSON53_04495 | SSON53_05165 | SSON53_05595 | SSON53_06535 | SSON53_06585 |
| SSON53_07385 | SSON53_07390 | SSON53_08640 | SSON53_08945 | SSON53_10840 | SSON53_12340 |
| pbpG | SSON53_13270 | SSON53_14190 | SSON53_14265 | SSON53_14460 | murP |
| SSON53_14705 | SSON53_14835 | SSON53_15125 | SSON53_16590 | SSON53_16955 | rumA |
| recD | SSON53_17670 | SSON53_17690 | SSON53_17705 | SSON53_17955 | SSON53_18080 |
| SSON53_18610 | SSON53_18735 | SSON53_18885 | SSON53_00360 | thiP | SSON53_00595 |
| SSON53_02980 | SSON53_03520 | SSON53_03560 | bioD | SSON53_04325 | SSON53_04710 |
| SSON53_04540 | SSON53_05585 | rne | SSON53_07205 | SSON53_08060 | SSON53_08955 |
| SSON53_08960 | SSON53_09205 | SSON53_10160 | SSON53_11100 | dacD | SSON53_13065 |
| fadJ | SSON53_14085 | SSON53_14455 | murQ | SSON53_16065 | SSON53_16755 |
| SSON53_18520 | SSON53_20320 | SSON53_21970 | SSON53_14020 | SSON53_24280 | SSON53_25150 |
| SSON53_25295 | SSON53_25400 | SSON53_00025 | SSON53_04285 | SSON53_04300 | SSON53_06415 |
| SSON53_07990 | SSON53_08770 | SSON53_09680 | SSON53_10655 | SSON53_11810 | SSON53_12330 |
| SSON53_12740 | SSON53_12795 | SSON53_13290 | SSON53_13415 | SSON53_13555 | SSON53_15280 |
| SSON53_16180 | SSON53_19150 | SSON53_22470 | SSON53_24775 | SSON53_24780 | SSON53_02695 |

**Table 14: List of genes**

| | | | | | |
|---|---|---|---|---|---|
| SSON53_16770 | SSON53_19730 | SSON53_14680 | SSON53_09620 | SSON53_25600 | SSON53_22935 |
| SSON53_24790 | SSON53_13530 | SSON53_12100 | SSON53_12655 | SSON53_04070 | SSON53_21285 |
| SSON53_01970 | wcaD | SSON53_08660 | SSON53_14430 | SSON53_17515 | SSON53_11020 |
| SSON53_00015 | SSON53_01080 | SSON53_12830 | SSON53_13420 | SSON53_13780 | SSON53_16000 |
| SSON53_16880 | SSON53_18540 | SSON53_22785 | SSON53_24145 | SSON53_25200 | SSON53_25460 |
| SSON53_03770 | SSON53_18890 | SSON53_16535 | SSON53_19285 | SSON53_03650 | SSON53_15285 |
| SSON53_18775 | SSON53_17120 | SSON53_24410 | SSON53_26285 | SSON53_14025 | SSON53_07220 |
| SSON53_08070 | SSON53_17780 | gcvT | SSON53_20935 | SSON53_21860 | SSON53_12595 |
| SSON53_04605 | SSON53_07910 | SSON53_13390 | queF | SSON53_18505 | pdxA |
| SSON53_00645 | SSON53_01515 | SSON53_02505 | SSON53_03060 | dpiB | SSON53_03230 |
| SSON53_03255 | SSON53_04495 | SSON53_05165 | SSON53_05595 | SSON53_06535 | SSON53_06585 |
| SSON53_07385 | SSON53_07390 | SSON53_08640 | SSON53_08945 | SSON53_10840 | SSON53_12340 |
| SSON53_02695 | SSON53_13270 | SSON53_14190 | SSON53_14265 | SSON53_14460 | murP |
| SSON53_14705 | SSON53_14835 | SSON53_15125 | SSON53_16590 | SSON53_16955 | SSON53_22470 |
| SSON53_24780 | SSON53_17670 | SSON53_17690 | SSON53_17705 | SSON53_17955 | SSON53_18080 |
| SSON53_18610 | SSON53_18735 | SSON53_18885 | SSON53_00360 | thiP | SSON53_00595 |
| SSON53_02980 | SSON53_03520 | SSON53_03560 | bioD | SSON53_04325 | SSON53_04710 |
| SSON53_04540 | SSON53_05585 | rne | SSON53_07205 | SSON53_08060 | SSON53_08955 |
| SSON53_08960 | SSON53_09205 | SSON53_10160 | SSON53_11100 | SSON53_24775 | SSON53_13065 |
| fadJ | SSON53_14085 | SSON53_14455 | murQ | SSON53_16065 | SSON53_16755 |
| SSON53_18520 | SSON53_20320 | SSON53_21970 | SSON53_14020 | SSON53_24280 | SSON53_25150 |
| SSON53_25295 | SSON53_25400 | SSON53_00025 | SSON53_04285 | SSON53_04300 | SSON53_06415 |
| SSON53_07990 | SSON53_08770 | SSON53_09680 | SSON53_10655 | SSON53_11810 | SSON53_12330 |
| SSON53_12740 | SSON53_12795 | SSON53_13290 | SSON53_13415 | SSON53_13555 | SSON53_15280 |
| SSON53_16180 | SSON53_19150 | | | | |

Furthermore, the following is described:
[1] A diagnostic method of determining an infection of a patient with Shigella species potentially resistant to antimicrobial drug, e.g. antibiotic, treatment, comprising the steps of:
   a) obtaining or providing a sample containing or suspected of containing at least one Shigella species from the patient;
   b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of an infection with an antimicrobial drug, e.g. antibiotic, resistant Shigella strain in said patient.
[2] A method of selecting a treatment of a patient suffering from an infection with a potentially resistant Shigella strain, comprising the steps of:
   a) obtaining or providing a sample containing or suspected of containing at least one Shigella species from the patient;
   b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of a resistance to one or more antimicrobial, e.g. antibiotic, drugs;
   c) identifying said at least one or more antimicrobial, e.g. antibiotic, drugs; and
   d) selecting one or more antimicrobial, e.g. antibiotic, drugs different from the ones identified in step c) and being suitable for the treatment of a Shigella infection.
[3] The method of [1] or [2], where the method involves determining the resistance of Shigella to one or more antimicrobial, e.g. antibiotic, drugs.
[4] The method of any one of [1] to [3], wherein the antimicrobial, e.g. antibiotic, drug is selected from lactam antibiotics and the presence of a mutation in the following genes is determined: SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400,SSON53_13555, SSON53_14945, SSON53_15285, fucI, SSON53_02755, SSON53_06415, SSON53_04615, SSON53_17330, and/or pdxA, or SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and/or SSON53_06085.
[5] The method of any one of [1] to [3], wherein the antimicrobial, e.g. antibiotic, drug is selected from polyketide antibiotics, preferably tetracycline antibiotics, and the presence of a mutation in the following genes is determined: metH, SSON53_24790, SSON53_08770, SSON53_24780, SSON53_15280, SSON53_10655, SSON53_13555, and/or SSON53_06415, or metH, SSON53_24790, SSON53_08770, SSON53_24780, SSON53_15280, SSON53_10655, SSON53_13555, and/or SSON53_06415.
[6] The method of one or more of [1] to [5], wherein the antimicrobial drug, e.g. antibiotic drug, is selected from the group consisting of Amoxicillin/K Clavulanate (AUG), Ampicillin (AM), Aztreonam (AZT), Cefazolin (CFZ), Cefepime (CPM), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).
[7] The method of any one of [1] to [6], wherein the antibiotic drug is CF and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
   wherein the antibiotic drug is CFZ and a mutation in at least one of the following nucleotide positions is detected with regard to reference genome NC_016822 as annotated at the NCBI: 3134050; and/or
   wherein the antibiotic drug is A/S and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
   wherein the antibiotic drug is CRM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
   wherein the antibiotic drug is P/T and a mutation in at least one of the following nucleotide positions is detected with regard to reference genome NC_016822 as annotated at the NCBI: 2241215, 4595667, 4502174, 2543074, 1159191, 2325584, 4707429, 3134050, 1655745, 949314, 1427818, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or wherein the antibiotic drug is AM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
   wherein the antibiotic drug is AUG and a mutation in at least one of the following nucleotide positions is detected with regard to reference genome NC_016822 as annotated at the NCBI: 2309519, 2314492, 4595667, 87512, 1159191, 2325584, 4707429, 3134050, 1427818, 2636621, 1429397, 2877526, 1200860; and/or
   wherein the antibiotic drug is TE and a mutation in at least one of the following nucleotide positions is detected with regard to reference genome NC_016822 as annotated at the NCBI: 4595667, 4707429, 1655745, 4705326, 2876708, 1990704, 2539010, 1257335.
[8] The method of any one of [1] to [7], wherein the resistance of a bacterial microorganism belonging to the species Shigella against 1, 2, 3, 4, 5, 6, 7, 8, 9, 10, 11, 12, 13, 14, 15 or 16, 17, 18, 19, 20 or 21 antibiotic drugs is determined.
[9] The method of one or more of [1] to [8], wherein determining the nucleic acid sequence information or the presence of a mutation comprises determining a partial sequence or an entire sequence of the at least two genes.
[10] The method of one or more of [1] to [9], wherein determining the nucleic acid sequence information or the presence of a mutation comprises determining a partial or entire sequence of the genome of the Shigella species, wherein said partial or entire sequence of the genome comprises at least a partial sequence of said at least two genes.
[11] The method of one or more of [1] to [10], wherein determining the nucleic acid sequence information or the presence of a mutation comprises using a next generation sequencing or high throughput sequencing method, preferably wherein a partial or entire genome sequence of the bacterial organism of Shigella species is determined by using a next generation sequencing or high throughput sequencing method.
[12] A method of determining an antimicrobial drug, e.g. antibiotic, resistance profile for bacterial microorganisms of Shigella species, comprising:
   obtaining or providing a first data set of gene sequences of a plurality of clinical isolates of Shigella species;
   providing a second data set of antimicrobial drug, e.g. antibiotic, resistance of the plurality of clinical isolates of Shigella species;
   aligning the gene sequences of the first data set to at least one, preferably one, reference genome of Shigella, or
   assembling the gene sequence of the first data set, at least in part;
   analyzing the gene sequences of the first data set for genetic variants to obtain a third data set of genetic variants;
   correlating the third data set with the second data set and statistically analyzing the correlation; and
   determining the genetic sites in the genome of Shigella associated with antimicrobial drug, e.g. antibiotic, resistance.
[13] A diagnostic method of determining an infection of a patient with Shigella species potentially resistant to antimicrobial drug treatment, comprising the steps of:
   a) obtaining or providing a sample containing or suspected of containing a bacterial microorganism belonging to the species Shigella from the patient;
   b) determining the presence of at least one mutation in at least one gene of the bacterial microorganism belonging to the species Shigella as determined by the method of [12], wherein the presence of said at least one mutation is indicative of an infection with an antimicrobial drug resistant Shigella strain in said patient.
[14] A method of selecting a treatment of a patient suffering from an infection with a potentially resistant Shigella strain, comprising the steps of:
   a) obtaining or providing a sample containing or suspected of containing a bacterial microorganism belonging to the species Shigella from the patient;
   b) determining the presence of at least one mutation in at least one gene of the bacterial microorganism belonging to the species Shigella as determined by the method of [12], wherein the presence of said at least one mutation is indicative of a resistance to one or more antimicrobial drugs;
   c) identifying said at least one or more antimicrobial drugs; and
   d) selecting one or more antimicrobial drugs different from the ones identified in step c) and being suitable for the treatment of a Shigella infection.
[15] A method of acquiring an antimicrobial drug, e.g. antibiotic, resistance profile for bacterial microorganisms of Shigella species, comprising:
   obtaining or providing a first data set of gene sequences of a clinical isolate of Shigella species;
   providing a second data set of antimicrobial drug, e.g. antibiotic, resistance of a plurality of clinical isolates of Shigella species;
   aligning the gene sequences of the first data set to at least one, preferably one, reference genome of Shigella, or
   assembling the gene sequence of the first data set, at least in part;
   analyzing the gene sequences of the first data set for genetic variants to obtain a third data set of genetic variants of the first data set;
   correlating the third data set with the second data set and statistically analyzing the correlation; and
   determining the genetic sites in the genome of Shigella of the first data set associated with antimicrobial drug, e.g. antibiotic, resistance.
[16] Computer program product comprising computer executable instructions which, when executed, perform a method according to any one of [13] to [15].

### Examples

The present invention will now be described in detail with reference to several examples thereof. However, these examples are illustrative and do not limit the scope of the invention.

### Example 1

Whole genome sequencing was carried out in addition to classical antimicrobial susceptibility testing of the same isolates for a cohort of 470 specimens. This allowed performing genome wide correlation studies to find genetic variants (e.g. point mutations, small insertions and deletion, larger structural variants, plasmid copy number gains, gene dosage effects) in the genome and plasmids that are significantly correlated to the resistance against one or several drugs. The approach also allows for comparing the relevant sites in the genome to each other.

In the approach the different sources of genetic resistance as well as the different ways of how bacteria can become resistant were covered. By measuring clinical isolates collected in a broad geographical area and across a broad time span of three decades a complete picture going far beyond the rather artificial step of laboratory generated resistance mechanisms was tried to be generated.

To this end, a set of 21 clinically relevant antimicrobial agents with 5 different modes of action was put together, and the minimally inhibitory concentration (MIC) of the 21 drugs for the Shigella isolates was measured.

The detailed procedure is given in the following:

### Bacterial Strains

The inventors selected 470 Shigella strains from the microbiology strain collection at Siemens Healthcare Diagnostics (West Sacramento, CA) for susceptibility testing and whole genome sequencing.

### Antimicrobial Susceptibility Testing (AST) Panels

Frozen reference AST panels were prepared following Clinical Laboratory Standards Institute (CLSI) recommendations. The following antimicrobial agents (with µg/ml concentrations shown in parentheses) were included in the panels: Amoxicillin/K Clavulanate (0.5/0.25-64/32), Ampicillin (0.25-128), Ampicillin/Sulbactam (0.5/0.25-64/32), Aztreonam (0.25-64), Cefazolin (0.5-32), Cefepime (0.25-64), Cefotaxime (0.25-128), Ceftazidime (0.25-64), Ceftriaxone (0.25-128), Cefuroxime (1-64), Cephalothin (1-64), Ciprofloxacin (0.015-8), Ertepenem (0.12-32), Gentamicin (0.12-32), Imipenem (0.25-32), Levofloxacin (0.25-16), Meropenem (0.12-32), Piperacillin/Tazobactam (0.25/4-256/4), Tetracycline (0.5-64), Tobramycin (0.12-32), and Trimethoprim/Sulfamethoxazole (0.25/4.7-32/608). Prior to use with clinical isolates, AST panels were tested with QC strains. AST panels were considered acceptable for testing with clinical isolates when the QC results met QC ranges described by CLSI16.

### Inoculum Preparation

Isolates were cultured on trypticase soy agar with 5% sheep blood (BBL, Cockeysville, Md.) and incubated in ambient air at 35±1°C for 18-24 h. Isolated colonies (4-5 large colonies or 5-10 small colonies) were transferred to a 3 ml Sterile Inoculum Water (Siemens) and emulsified to a final turbidity of a 0.5 McFarland standard. 2 ml of this suspension was added to 25 ml Inoculum Water with Pluronic-F (Siemens). Using the Inoculator (Siemens) specific for frozen AST panels, 5 µl of the cell suspension was transferred to each well of the AST panel. The inoculated AST panels were incubated in ambient air at 35±1°C for 16-20 h. Panel results were read visually, and minimal inhibitory concentrations (MIC) were determined.

### DNA extraction

Four streaks of each Gram-negative bacterial isolate cultured on trypticase soy agar containing 5% sheep blood and cell suspensions were made in sterile 1.5 ml collection tubes containing 50 µl Nuclease-Free Water (AM9930, Life Technologies). Bacterial isolate samples were stored at -20 °C until nucleic acid extraction. The Tissue Preparation System (TPS) (096D0382-02_01_B, Siemens) and the VERSANT® Tissue Preparation Reagents (TPR) kit (10632404B, Siemens) were used to extract DNA from these bacterial isolates. Prior to extraction, the bacterial isolates were thawed at room temperature and were pelleted at 2000 G for 5 seconds. The DNA extraction protocol DNAext was used for complete total nucleic acid extraction of 48 isolate samples and eluates, 50 µl each, in 4 hours. The total nucleic acid eluates were then transferred into 96-Well qPCR Detection Plates (401341, Agilent Technologies) for RNase A digestion, DNA quantitation, and plate DNA concentration standardization processes. RNase A (AM2271, Life Technologies) which was diluted in nuclease-free water following manufacturer's instructions was added to 50 µl of the total nucleic acid eluate for a final working concentration of 20 µg/ml. Digestion enzyme and eluate mixture were incubated at 37°C for 30 minutes using Siemens VERSANT® Amplification and Detection instrument. DNA from the RNase digested eluate was quantitated using the Quant-iT™ PicoGreen dsDNA Assay (P11496, Life Technologies) following the assay kit instruction, and fluorescence was determined on the Siemens VERSANT® Amplification and Detection instrument. Data analysis was performed using Microsoft® Excel 2007. 25 µl of the quantitated DNA eluates were transferred into a new 96-Well PCR plate for plate DNA concentration standardization prior to library preparation. Elution buffer from the TPR kit was used to adjust DNA concentration. The standardized DNA eluate plate was then stored at -80°C until library preparation.

### Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequncing chemistry (Illumina). Basic sequencing quality parameters were determined using the FastQC quality control tool for high throughput sequence data (Babraham Bioinformatics Institute).

### Data analysis

Raw paired-end sequencing data for the 470 Shigella samples were mapped against the Shigella reference (NC_016822) with BWA 0.6.1.20. The resulting SAM files were sorted, converted to BAM files, and PCR duplicates were marked using the Picard tools package 1.104 (http://picard.sourceforge.net/). The Genome Analysis Toolkit 3.1.1 (GATK)21 was used to call SNPs and indels for blocks of 200 Shigella samples (parameters: - ploidy 1 -glm BOTH -stand_call_conf 30 -stand_emit_conf 10). VCF files were combined into a single file and quality filtering for SNPs was carried out (QD < 2.0 | | FS > 60.0 | | MQ < 40.0) and indels (QD < 2.0 | | FS > 200.0). Detected variants were annotated with SnpEff22 to predict coding effects. For each annotated position, genotypes of all Shigella samples were considered. Shigella samples were split into two groups, low resistance group (having lower MIC concentration for the considered drug), and high resistance group (having higher MIC concentrations) with respect to a certain MIC concentration (breakpoint). To find the best breakpoint all thresholds were evaluated and p-values were computed with Fisher's exact test relying on a 2x2 contingency table (number of Shigella samples having the reference or variant genotype vs. number of samples belonging to the low and high resistance group). The best computed breakpoint was the threshold yielding the lowest p-value for a certain genomic position and drug. For further analyses positions with non-synonymous alterations and p-value < 10⁻⁹ were considered.

Since a potential reason for drug resistance is gene duplication, gene dose dependency was evaluated. For each sample the genomic coverage for each position was determined using BED Tools. 23 Gene ranges were extracted from the reference assembly NC_010473.gff and the normalized median coverage per gene was calculated. To compare low- and high-resistance isolates the best area under the curve (AUC) value was computed. Groups of at least 20% of all samples having a median coverage larger than zero for that gene and containing more than 15 samples per group were considered in order to exclude artifacts and cases with AUC > 0.75 were further evaluated.

To include data on the different ways how resistance mechanisms are acquired Shigella isolates collected over more than three decades were analyzed such that also horizontal gene transfer could potentially be discovered.

In detail, the following steps were carried out:
Shigella strains to be tested were seeded on agar plates and incubated under growth conditions for 24 hours. Then, colonies were picked and incubated in growth medium in the presence of a given antibiotic drug in dilution series under growth conditions for 16-20 hours. Bacterial growth was determined by observing turbidity.

Next mutations were searched that are highly correlated with the results of the phenotypic resistance test.

For sequencing, samples were prepared using a Nextera library preparation, followed by multiplexed sequencing using the Illuminat HiSeq 2500 system, paired end sequencing. Data were mapped with BWA (Li H. and Durbin R. (2010) Fast and accurate long-read alignment with Burrows-Wheeler Transform. Bioinformatics, Epub. [PMID: 20080505])and SNP were called using samtools (Li H.*, Handsaker B.*, Wysoker A., Fennell T., Ruan J., Homer N., Marth G., Abecasis G., Durbin R. and 1000 Genome Project Data Processing Subgroup (2009) The Sequence alignment/map (SAM) format and SAMtools. Bioinformatics, 25, 2078-9. [PMID: 19505943]).

As reference genome, NC_016822 as annotated at the NCBI was determined as best suited.

The mutations were matched to the genes and the amino acid changes were calculated. Using different algorithms (SVM, homology modeling) mutations leading to amino acid changes with likely pathogenicity / resistance were calculated.

In total, whole genomes and plasmids of about 470 different clinical isolates of Shigella species were sequenced, and classical antimicrobial susceptibility testing (AST) against 21 therapy forms as described above was performed for all organisms. From the classical AST a table with 470 rows (isolates) and 21 columns (MIC values for 21 drugs) was obtained. Each table entry contained the MIC for the respective isolate and the respective drug. The genetic data were mapped to different reference genomes of Shigella that have been annotated at the NCBI (http://www.ncbi.nlm.nih.gov/), and the best reference was chosen as template for the alignment - NC_016822 as annotated at the NCBI. Additionally, assemblies were carried out and it was verified that the sequenced genomes fulfil all quality criteria to become reference genomes.

Next, genetic variants were evaluated. This approach resulted in a table with about five millions genetic sites in columns and the same isolates in 470 rows. Each table entry contained the genetic determinant at the respective site (A, C, T, G, small insertions and deletions, ...) for the respective isolate.

In a next step different statistical tests were carried out
1) For comparing resistance / susceptibility to genetic sites we calculated contingency tables and determined the significance using Fishers test
2) For comparing different sites to each other we calculated the correlation between different genetic sites
3) For detecting gene dosage effects, e.g. loss or gain of genes (in the genome or on plasmids) we calculated the coverage (i.e. how many read map to the current position) at each site for resistant and not resistant isolates.

From the data, first the 50 genes with the best p-value were chosen for the list of mutations as well as the list of correlated antibiotic resistance, representing Tables 1 and 2. As can be seen from Tables 1 and 2, slight differences between the tables can be observed, showing the necessity to carry out both steps for determining statistical significant data for antimicrobial drug, e.g. antibiotic, resistance profiles.

A full list of all genetic sites, drugs, drug classes, affected genes etc. is provided in Tables 3 and 4a, 4b and 4c, wherein Table 3 corresponds to Table 1 and represents the genes having the lowest p-values after determining mutations in the genes, and Table 4, respectively Tables 4a, 4b and 4c correspond to Table 2 and represent the genes having the lowest p-values after correlating the mutations with antibiotic resistance.

**Table 3: Detailed results for the genes in Example 1 (corresponding to Table 1)**

| **POS** | **drug class** | **#drug classes** | **p**-**value** | **gene name** | **genbank protein accession number** |
|---|---|---|---|---|---|
| 2241215 | Lactams | 1 | 7,985E-51 | SSON53_12105 | YP_005456953.1 |
| 2309519 | Lactams | 1 | 2,5048E-49 | SSON53_12455 | YP_005457021.1 |
| 2233949 | Lactams | 1 | 8,7825E-49 | SSON53_12070 | YP_005456946.1 |
| 2314492 | Lactams | 1 | 1,9851E-48 | SSON53_12475 | YP_005457025.1 |
| 4595667 | polyketide (tetracycline);Lactams | 2 | 2,2331E-47 | metH | YP_005459323.1 |
| 87512 | Lactams | 1 | 6,3481E-47 | SSON53_00425 | YP_005454674.1 |
| 4502174 | Lactams | 1 | 6,6005E-47 | SSON53_23830 | YP_005459252.1 |
| 2543074 | Lactams | 1 | 1,1364E-46 | SSON53_13575 | YP_005457242.1 |
| 1159191 | Lactams | 1 | 1,4615E-46 | flgE | YP_005455738.1 |
| 2325584 | Lactams | 1 | 1,3919E-46 | SSON53_12500 | YP_005457030.1 |
| 1303652 | Lactams | 1 | 2,061E-46 | SSON53_06660 | YP_005455888.1 |
| 4707429 | polyketide (tetracycline);Lactams | 2 | 2,4042E-46 | SSON53_24790 | YP_005459432.1 |
| 3134050 | Lactams | 1 | 2,9472E-46 | SSON53_16770 | YP_005457860.1 |
| 4648709 | Lactams | 1 | 3,2112E-46 | SSON53_24480 | YP_005459374.1 |
| 1655745 | polyketide (tetracycline);Lactams | 2 | 4,5874E-46 | SSON53_08770 | YP_005456302.1 |
| 949314 | Lactams | 1 | 5,682E-46 | SSON53_04795 | YP_005455533.1 |
| 1427818 | Lactams | 1 | 6,4885E-46 | SSON53_07430 | YP_005456040.1 |
| 2636621 | Lactams | 1 | 6,8061E-46 | SSON53_14085 | YP_005457334.1 |
| 1429397 | Lactams | 1 | 7,2975E-46 | SSON53_07440 | YP_005456042.1 |
| 1690889 | Lactams | 1 | 8,2598E-46 | SSON53_08950 | YP_005456338.1 |
| 4856482 | Lactams | 1 | 8,0552E-46 | SSON53_25555 | YP_005459583.1 |
| 1723070 | Lactams | 1 | 1,0803E-45 | SSON53_09145 | YP_005456375.1 |
| 4857482 | Lactams | 1 | 9,8832E-46 | SSON53_25565 | YP_005459585.1 |
| 4705326 | polyketide (tetracycline);Lactams | 2 | 1,5221E-45 | SSON53_24780 | YP_005459430.1 |
| 862961 | Lactams | 1 | 2,1553E-45 | SSON53_04400 | YP_005455454.1 |
| 2876708 | polyketide (tetracycline);Lactams | 2 | 2,2052E-45 | SSON53_15280 | YP_005457566.1 |
| 887353 | Lactams | 1 | 2,3982E-45 | rimO | YP_005455475.1 |
| 980321 | Lactams | 1 | 2,4252E-45 | SSON53_04930 | YP_005455560.1 |
| 4429947 | Lactams | 1 | 2,6598E-45 | SSON53_23390 | YP_005459166.1 |
| 4431976 | Lactams | 1 | 2,6598E-45 | rhaB | YP_005459168.1 |
| 3376607 | Lactams | 1 | 2,8513E-45 | SSON53_17960 | YP_005458096.1 |
| 4565385 | Lactams | 1 | 2,9399E-45 | thiH | YP_005459296.1 |
| 750613 | Lactams | 1 | 3,6928E-45 | SSON53_03725 | YP_005455321.1 |
| 1781189 | Lactams | 1 | 3,71E-45 | SSON53_09500 | YP_005456446.1 |
| 4821412 | Lactams | 1 | 3,7289E-45 | SSON53_25405 | YP_005459553.1 |
| 2533864 | Lactams | 1 | 4,2097E-45 | SSON53_13530 | YP_005457233.1 |
| 1511130 | Lactams | 1 | 8,5623E-45 | astD | YP_005456138.1 |
| 1513422 | Lactams | 1 | 8,5623E-45 | SSON53_07945 | YP_005456141.1 |
| 1901825 | Lactams | 1 | 8,1722E-45 | SSON53_10080 | YP_005456556.1 |
| 1990704 | polyketide (tetracycline);Lactams | 2 | 8,344E-45 | SSON53_10655 | YP_005456671.1 |
| 2506615 | Lactams | 1 | 7,2324E-45 | SSON53_13400 | YP_005457207.1 |
| 2539010 | polyketide (tetracycline);Lactams | 2 | 7,7328E-45 | SSON53_13555 | YP_005457238.1 |
| 2803779 | Lactams | 1 | 7,875E-45 | SSON53_14945 | YP_005457501.1 |
| 2877526 | Lactams | 1 | 7,2573E-45 | SSON53_15285 | YP_005457567.1 |
| 3239464 | Lactams | 1 | 8,2293E-45 | fucI | YP_005457971.1 |
| 545532 | Lactams | 1 | 9,0371E-45 | SSON53_02755 | YP_005455128.1 |
| 1257335 | polyketide (tetracycline);Lactams | 2 | 8,9271E-45 | SSON53_06415 | YP_005455839.1 |
| 906989 | Lactams | 1 | 9,9901E-45 | SSON53_04615 | YP_005455497.1 |
| 3237959 | Lactams | 1 | 1,0949E-44 | SSON53_17330 | YP_005457970.1 |
| 60014 | Lactams | 1 | 1,307E-44 | pdxA | YP_005454652.1 |

**Table 4a: Detailed results for the genes in Example 1 (corresponding to Table 2)**

| **POS** | **drug** | **#drugs** | **drug class** | **#drug classes** |
|---|---|---|---|---|
| 2241215 | CF;A/S;CRM;P/T;AM | 5 | Lactams | 1 |
| 2309519 | CF;A/S;CRM;P/T;AM;AUG | 6 | Lactams | 1 |
| 2314492 | CF;A/S;CRM;AM;AUG | 5 | Lactams | 1 |
| 4595667 | CF;TE;A/S;CRM;P/T;AM;AUG | 7 | Polyketide*;Lactams | 2 |
| 87512 | CF;A/S;CRM;AM;AUG | 5 | Lactams | 1 |
| 4502174 | CF;A/S;CRM;P/T;AM | 5 | Lactams | 1 |
| 2543074 | CF;A/S;CRM;P/T;AM | 5 | Lactams | 1 |
| 1159191 | CF;A/S;CRM;P/T;AM;AUG | 6 | Lactams | 1 |
| 2325584 | CF;A/S;CRM;P/T;AM;AUG | 6 | Lactams | 1 |
| 4707429 | CF;TE;A/S;CRM;P/T;AM;AUG | 7 | Polyketide*;Lactams | 2 |
| 3134050 | CF;CFZ;CRM;P/T;AM;A/S;AUG | 7 | Lactams | 1 |
| 1655745 | CF;TE;A/S;CRM;P/T;AM | 6 | Polyketide*;Lactams | 2 |
| 949314 | CF;A/S;CRM;P/T;AM | 5 | Lactams | 1 |
| 1427818 | CF;A/S;CRM;P/T;AM;AUG | 6 | Lactams | 1 |
| 2636621 | CF;A/S;CRM;AM;AUG | 5 | Lactams | 1 |
| 1429397 | CF;A/S;CRM;P/T;AM;AUG | 6 | Lactams | 1 |
| 1690889 | CF;A/S;CRM;P/T;AM | 5 | Lactams | 1 |
| 4856482 | CF;A/S;CRM;P/T;AM | 5 | Lactams | 1 |
| 1723070 | CF;A/S;CRM;P/T;AM | 5 | Lactams | 1 |
| 4857482 | CF;A/S;CRM;P/T;AM | 5 | Lactams | 1 |
| 4705326 | CF;TE;A/S;CRM;P/T;AM | 6 | Polyketide*;Lactams | 2 |
| 862961 | CF;A/S;CRM;P/T;AM | 5 | Lactams | 1 |
| 2876708 | CF;TE;A/S;CRM;P/T;AM | 6 | Polyketide*;Lactams | 2 |
| 980321 | CF;A/S;CRM;P/T;AM | 5 | Lactams | 1 |
| 4429947 | CF;A/S;CRM;P/T;AM | 5 | Lactams | 1 |
| 4431976 | CF;A/S;CRM;P/T;AM | 5 | Lactams | 1 |
| 3376607 | CF;A/S;CRM;P/T;AM | 5 | Lactams | 1 |
| 4565385 | CF;A/S;CRM;P/T;AM | 5 | Lactams | 1 |
| 750613 | CF;A/S;CRM;P/T;AM | 5 | Lactams | 1 |
| 1781189 | CF;A/S;CRM;P/T;AM | 5 | Lactams | 1 |
| 4821412 | CF;A/S;CRM;P/T;AM | 5 | Lactams | 1 |
| 2533864 | CF;A/S;CRM;P/T;AM | 5 | Lactams | 1 |
| 1511130 | CF;A/S;CRM;P/T;AM | 5 | Lactams | 1 |
| 1513422 | CF;A/S;CRM;P/T;AM | 5 | Lactams | 1 |
| 1901825 | CF;A/S;CRM;P/T;AM | 5 | Lactams | 1 |
| 1990704 | CF;TE;A/S;CRM;P/T;AM | 6 | Polyketide*;Lactams | 2 |
| 2506615 | CF;A/S;CRM;P/T;AM | 5 | Lactams | 1 |
| 2539010 | CF;TE;A/S;CRM;P/T;AM | 6 | polyketide*;Lactams | 2 |
| 2803779 | CF;A/S;CRM;P/T;AM | 5 | Lactams | 1 |
| 2877526 | CF;A/S;CRM;P/T;AM;AUG | 6 | Lactams | 1 |
| 3239464 | CF;A/S;CRM;P/T;AM | 5 | Lactams | 1 |
| 545532 | CF;A/S;CRM;P/T;AM | 5 | Lactams | 1 |
| 1257335 | CF;TE;A/S;CRM;P/T;AM | 6 | polyketide*;Lactams | 2 |
| 906989 | CF;A/S;CRM;P/T;AM | 5 | Lactams | 1 |
| 3237959 | CF;A/S;CRM;P/T;AM | 5 | Lactams | 1 |
| 60014 | CF;A/S;CRM;P/T;AM | 5 | Lactams | 1 |
| 921872 | CF;A/S;CRM;P/T;AM | 5 | Lactams | 1 |
| 983367 | CF;A/S;CRM;P/T;AM | 5 | Lactams | 1 |
| 1182426 | CF;A/S;CRM;P/T;AM | 5 | Lactams | 1 |
| 1200860 | CF;A/S;CRM;P/T;AM;AUG | 6 | Lactams | 1 |

| | | | | |
|---|---|---|---|---|
| *: (tetracycline) | | | | |

**Table 4b: Detailed results for the genes in Example 1 (corresponding to Table 2, continued)**

| **POS** | **best drug** | **#significant Lactams** | **#significant fluoroquinolones** | **#significant aminoglycosides** | **#significant polyketide (tetracycline)** | **#significant other (benzene derived)/ sulfonamide** |
|---|---|---|---|---|---|---|
| 2241215 | CRM | 5 | 0 | 0 | 0 | 0 |
| 2309519 | CF | 6 | 0 | 0 | 0 | 0 |
| 2314492 | CF | 5 | 0 | 0 | 0 | 0 |
| 4595667 | CRM | 6 | 0 | 0 | 1 | 0 |
| 87512 | CF | 5 | 0 | 0 | 0 | 0 |
| 4502174 | CRM | 5 | 0 | 0 | 0 | 0 |
| 2543074 | CRM | 5 | 0 | 0 | 0 | 0 |
| 1159191 | CRM | 6 | 0 | 0 | 0 | 0 |
| 2325584 | CF | 6 | 0 | 0 | 0 | 0 |
| 4707429 | CRM | 6 | 0 | 0 | 1 | 0 |
| 3134050 | CRM | 7 | 0 | 0 | 0 | 0 |
| 1655745 | CRM | 5 | 0 | 0 | 1 | 0 |
| 949314 | CRM | 5 | 0 | 0 | 0 | 0 |
| 1427818 | CRM | 6 | 0 | 0 | 0 | 0 |
| 2636621 | CF | 5 | 0 | 0 | 0 | 0 |
| 1429397 | CRM | 6 | 0 | 0 | 0 | 0 |
| 1690889 | CRM | 5 | 0 | 0 | 0 | 0 |
| 4856482 | CRM | 5 | 0 | 0 | 0 | 0 |
| 1723070 | CRM | 5 | 0 | 0 | 0 | 0 |
| 4857482 | CRM | 5 | 0 | 0 | 0 | 0 |
| 4705326 | CRM | 5 | 0 | 0 | 1 | 0 |
| 862961 | CRM | 5 | 0 | 0 | 0 | 0 |
| 2876708 | CRM | 5 | 0 | 0 | 1 | 0 |
| 980321 | CRM | 5 | 0 | 0 | 0 | 0 |
| 4429947 | CRM | 5 | 0 | 0 | 0 | 0 |
| 4431976 | CRM | 5 | 0 | 0 | 0 | 0 |
| 3376607 | CRM | 5 | 0 | 0 | 0 | 0 |
| 4565385 | CRM | 5 | 0 | 0 | 0 | 0 |
| 750613 | CRM | 5 | 0 | 0 | 0 | 0 |
| 1781189 | CRM | 5 | 0 | 0 | 0 | 0 |
| 4821412 | CRM | 5 | 0 | 0 | 0 | 0 |
| 2533864 | CRM | 5 | 0 | 0 | 0 | 0 |
| 1511130 | CRM | 5 | 0 | 0 | 0 | 0 |
| 1513422 | CRM | 5 | 0 | 0 | 0 | 0 |
| 1901825 | CRM | 5 | 0 | 0 | 0 | 0 |
| 1990704 | CRM | 5 | 0 | 0 | 1 | 0 |
| 2506615 | CRM | 5 | 0 | 0 | 0 | 0 |
| 2539010 | CRM | 5 | 0 | 0 | 1 | 0 |
| 2803779 | CRM | 5 | 0 | 0 | 0 | 0 |
| 2877526 | CRM | 6 | 0 | 0 | 0 | 0 |
| 3239464 | CRM | 5 | 0 | 0 | 0 | 0 |
| 545532 | CRM | 5 | 0 | 0 | 0 | 0 |
| 1257335 | CRM | 5 | 0 | 0 | 1 | 0 |
| 906989 | CRM | 5 | 0 | 0 | 0 | 0 |
| 3237959 | CRM | 5 | 0 | 0 | 0 | 0 |
| 60014 | CRM | 5 | 0 | 0 | 0 | 0 |
| 921872 | CRM | 5 | 0 | 0 | 0 | 0 |
| 983367 | CRM | 5 | 0 | 0 | 0 | 0 |
| 1182426 | CRM | 5 | 0 | 0 | 0 | 0 |
| 1200860 | CF | 6 | 0 | 0 | 0 | 0 |

**Table 4c: Detailed results for the genes in Example 1 (corresponding to Table 2, continued)**

| **POS** | **p**-**value** | **gene name** | **genbank protein accession number** |
|---|---|---|---|
| 2241215 | 2,29978E-44 | SSON53_12105 | YP_005456953.1 |
| 2309519 | 3,60701E-43 | SSON53_12455 | YP_005457021.1 |
| 2314492 | 1,4137E-42 | SSON53_12475 | YP_005457025.1 |
| 4595667 | 9,18813E-42 | metH | YP_005459323.1 |
| 87512 | 1,90101E-41 | SSON53_00425 | YP_005454674.1 |
| 4502174 | 1,90101E-41 | SSON53_23830 | YP_005459252.1 |
| 2543074 | 2,97533E-41 | SSON53_13575 | YP_005457242.1 |
| 1159191 | 3,23782E-41 | flgE | YP_005455738.1 |
| 2325584 | 3,23782E-41 | SSON53_12500 | YP_005457030.1 |
| 4707429 | 4,32775E-41 | SSON53_24790 | YP_005459432.1 |
| 3134050 | 4,99312E-41 | SSON53_16770 | YP_005457860.1 |
| 1655745 | 6,60616E-41 | SSON53_08770 | YP_005456302.1 |
| 949314 | 7,79281E-41 | SSON53_04795 | YP_005455533.1 |
| 1427818 | 8,12502E-41 | SSON53_07430 | YP_005456040.1 |
| 2636621 | 8,16766E-41 | SSON53_14085 | YP_005457334.1 |
| 1429397 | 8,40704E-41 | SSON53_07440 | YP_005456042.1 |
| 1690889 | 8,49615E-41 | SSON53_08950 | YP_005456338.1 |
| 4856482 | 8,49615E-41 | SSON53_25555 | YP_005459583.1 |
| 1723070 | 9,15152E-41 | SSON53_09145 | YP_005456375.1 |
| 4857482 | 9,15152E-41 | SSON53_25565 | YP_005459585.1 |
| 4705326 | 1,25253E-40 | SSON53_24780 | YP_005459430.1 |
| 862961 | 1,71651E-40 | SSON53_04400 | YP_005455454.1 |
| 2876708 | 1,71651E-40 | SSON53_15280 | YP_005457566.1 |
| 980321 | 1,791E-40 | SSON53_04930 | YP_005455560.1 |
| 4429947 | 1,82392E-40 | SSON53_23390 | YP_005459166.1 |
| 4431976 | 1,82392E-40 | rhaB | YP_005459168.1 |
| 3376607 | 1,90979E-40 | SSON53_17960 | YP_005458096.1 |
| 4565385 | 1,91751E-40 | thiH | YP_005459296.1 |
| 750613 | 2,19179E-40 | SSON53_03725 | YP_005455321.1 |
| 1781189 | 2,19179E-40 | SSON53_09500 | YP_005456446.1 |
| 4821412 | 2,19179E-40 | SSON53_25405 | YP_005459553.1 |
| 2533864 | 2,37735E-40 | SSON53_13530 | YP_005457233.1 |
| 1511130 | 3,73641E-40 | astD | YP_005456138.1 |
| 1513422 | 3,73641E-40 | SSON53_07945 | YP_005456141.1 |
| 1901825 | 3,73641E-40 | SSON53_10080 | YP_005456556.1 |
| 1990704 | 3,73641E-40 | SSON53_10655 | YP_005456671.1 |
| 2506615 | 3,73641E-40 | SSON53_13400 | YP_005457207.1 |
| 2539010 | 3,73641E-40 | SSON53_13555 | YP_005457238.1 |
| 2803779 | 3,73641E-40 | SSON53_14945 | YP_005457501.1 |
| 2877526 | 3,73641E-40 | SSON53_15285 | YP_005457567.1 |
| 3239464 | 3,73641E-40 | fucI | YP_005457971.1 |
| 545532 | 3,82761E-40 | SSON53_02755 | YP_005455128.1 |
| 1257335 | 3,82761E-40 | SSON53_06415 | YP_005455839.1 |
| 906989 | 4,16996E-40 | SSON53_04615 | YP_005455497.1 |
| 3237959 | 4,20456E-40 | SSON53_17330 | YP_005457970.1 |
| 60014 | 4,28671E-40 | pdxA | YP_005454652.1 |
| 921872 | 4,28671E-40 | SSON53_04690 | YP_005455512.1 |
| 983367 | 4,28671E-40 | SSON53_04945 | YP_005455563.1 |
| 1182426 | 4,28671E-40 | SSON53_06005 | YP_005455761.1 |
| 1200860 | 4,28671E-40 | SSON53_06085 | YP_005455777.1 |

In addition, the data with the best p-values for each antibiotic class as well as each antibiotic, respectively, were evaluated, being disclosed in Tables 5 - 12.

In Tables 3 - 12 the columns are designated as follows:
Gene name: affected gene;
POS: genomic position of the SNP / variant in the Shigella reference genome (see above);
P-value: significance value calculated using fishers exact test;
genbank protein accession number: (NCBI) Accession number of the corresponding protein of the genes

Also the antibiotic/drug classes, the number of significant antibiotics correlated to the mutations (over all antibiotics or over certain classes), as well as the correlated antibiotics are denoted in the Tables.

The P-value was calculated using the fisher exact test based on contingency table with 4 fields: #samples Resistant / wild type; #samples Resistant / mutant; #samples not Resistant / wild type; #samples not Resistant / mutant

The test is based on the distribution of the samples in the 4 fields. Even distribution indicates no significance, while clustering into two fields indicates significance.

The following results were obtained
- A total of 49,560 different correlations between genetic sites and anti-microbial agents were detected (p-value < 10⁻⁶) .
- These refer to 35,686 unique genetic positions such that each position was significant in 1.388 drugs on average
- The biggest part of these were point mutations (i.e. single base exchanges)
- The highest significance (10⁻⁵⁰) was reached for a frameshift mutation in YP_005456953.1
- Besides these, insertions or deletions of up to four bases were discovered
- Further, potential genetic tests for five different drug classes relating to resistances were discovered
   - β**-**lactams (includes Penicillins, Cephalosporins, Carbapenems, Monobactams)
   - Quinolones, particularly Fluoroquinolones
   - Aminoglycosides
   - Polyketides, particularly Tetracyclines
   - Folate synthesis inhibitors
- Potential genetic tests for all tested drugs/drug combinations were discovered: Amoxicillin/Clavulanate, Ampicillin, Ampicillin/Sulbactam, Aztreonam, Cefazolin, Cefepime, Ceftazidime,Cefuroxime, Cephalothin, Imipenem, Piperacillin/Tazobactam, Ciprofloxacin, Levofloxacin, Gentamycin, Tobramycin, Tetracycline, Trimethoprim/Sulfamethoxazol
- Mutations were observed in 4,159 different genes

A genetic test for the combined pathogen identification and antimicrobial susceptibility testing direct from the patient sample can reduce the time-to actionable result significantly from several days to hours, thereby enabling targeted treatment. Furthermore, this approach will not be restricted to central labs, but point of care devices can be developed that allow for respective tests. Such technology along with the present methods and computer program products could revolutionize the care, e.g. in intense care units or for admissions to hospitals in general. Furthermore, even applications like real time outbreak monitoring can be achieved using the present methods.

Instead of using only single variants, a combination of several variant positions can improve the prediction accuracy and further reduce false positive findings that are influenced by other factors.

Compared to approaches using MALDI-TOF MS, the present approach has the advantage that it covers almost the complete genome and thus enables us to identify the potential genomic sites that might be related to resistance. While MALDI-TOF MS can also be used to identify point mutations in bacterial proteins, this technology only detects a subset of proteins and of these not all are equally well covered. In addition, the identification and differentiation of certain related strains is not always feasible.

The present method allows computing a best breakpoint for the separation of isolates into resistant and susceptible groups. The inventors designed a flexible software tool that allows to consider - besides the best breakpoints - also values defined by different guidelines (e.g. European and US guidelines), preparing for an application of the GAST in different countries.

The inventors demonstrate that the present approach is capable of identifying mutations in genes that are already known as drug targets, as well as detecting potential new target sites.

The current approach enables
a. Identification and validation of markers for genetic identification and susceptibility/resistance testing within one diagnostic test
b. validation of known drug targets and modes of action
c. detection of potentially novel resistance mechanisms leading to putative novel target / secondary target genes for new therapies

## Claims

1. A diagnostic method of determining an infection of a patient with Shigella species potentially resistant to antibiotic drug treatment, comprising the steps of:
a) obtaining or providing a sample containing or suspected of containing at least one Shigella species from the patient;
b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53 12500, SSON53_06660, SSON53_24790, SSON53_16770, SSON53_24480, SSON53_08770, SSON53_04795, SSON53_07430, SSON53_14085, SSON53_07440, SSON53_08950, SSON53_25555, SSON53 09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400,SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400,SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of an infection with an antibiotic drug resistant Shigella strain in said patient,
wherein one of the at least two genes is metH, the antibiotic drug is Cephalotin (CF), Cefazolin (CFZ), Ampicillin/Sulbactam (A/S), Cefuroxime (CRM), Piperacillin/Tazobactam (P/T), Ampicillin (AM), Amoxicillin/K Clavulanate (AUG) and/or Tetracycline (TE) and at least a mutation in nucleotide position 4595667 is detected with regard to reference genome NC_016822 as annotated at the NCBI, and
- wherein the antibiotic drug is CF and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is CFZ and a mutation in at least one of the following nucleotide positions is detected with regard to reference genome NC_016822 as annotated at the NCBI: 3134050; and/or
- wherein the antibiotic drug is A/S and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is CRM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is P/T and a mutation in at least one of the following nucleotide positions is detected with regard to reference genome NC_016822 as annotated at the NCBI: 2241215, 4595667, 4502174, 2543074, 1159191, 2325584, 4707429, 3134050, 1655745, 949314, 1427818, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is AM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is AUG and a mutation in at least one of the following nucleotide positions is detected with regard to reference genome NC_016822 as annotated at the NCBI: 2309519, 2314492, 4595667, 87512, 1159191, 2325584, 4707429, 3134050, 1427818, 2636621, 1429397, 2877526, 1200860; and/or
- wherein the antibiotic drug is TE and a mutation in at least one of the following nucleotide positions is detected with regard to reference genome NC_016822 as annotated at the NCBI: 4595667, 4707429, 1655745, 4705326, 2876708, 1990704, 2539010, 1257335.

2. A method of selecting a treatment of a patient suffering from an infection with a potentially resistant Shigella strain, comprising the steps of:
a) obtaining or providing a sample containing or suspected of containing at least one Shigella species from the patient;
b) determining the presence of at least one mutation in at least two genes from the group of genes consisting of SSON53_12105, SSON53_12455, SSON53_12070, SSON53_12475, metH, SSON53_00425, SSON53_23830, SSON53_13575, flgE, SSON53_12500, SSON53_06660, SSON53_24790, SSON53_16770, SSON53_24480, SSON53_08770, SSON53_04795, SSON53_07430, SSON53 14085, SSON53_07440, SSON53_08950, SSON53_25555, SSON53_09145, SSON53_25565, SSON53_24780, SSON53_04400, SSON53_15280, rimO, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400,SSON53_13555, SSON53_14945, SSON53_15285, fucI, SSON53_02755, SSON53_06415, SSON53_04615, SSON53_17330, and pdxA, or from the group of genes consisting of SSON53_12105, SSON53_12455, SSON53_12475, metH, SSON53_00425, SSON53_23830, SSON53_13575, flgE, SSON53_12500, SSON53_24790, SSON53_16770, SSON53_08770, SSON53_04795, SSON53_07430, SSON53_14085, SSON53_07440, SSON53_08950, SSON53_25555, SSON53_09145, SSON53_25565, SSON53_24780, SSON53_04400, SSON53_15280, SSON53_04930, SSON53_23390, rhaB, SSON53_17960, thiH, SSON53_03725, SSON53_09500, SSON53_25405, SSON53_13530, astD, SSON53_07945, SSON53_10080, SSON53_10655, SSON53_13400, SSON53_13555, SSON53_14945, SSON53_15285, fucI, SSON53_02755, SSON53_06415, SSON53_04615, SSON53_17330, pdxA, SSON53_04690, SSON53_04945, SSON53_06005, and SSON53_06085, wherein the presence of said at least two mutations is indicative of a resistance to one or more antibiotic drugs;
c) identifying said at least one or more antibiotic drugs; and
d) selecting one or more antibiotic drugs different from the ones identified in step c) and being suitable for the treatment of a Shigella infection,
wherein one of the at least two genes is metH, the antibiotic drug is Cephalotin (CF), Cefazolin (CFZ), Ampicillin/Sulbactam (A/S), Cefuroxime (CRM), Piperacillin/Tazobactam (P/T), Ampicillin (AM), Amoxicillin/K Clavulanate (AUG) and/or Tetracycline (TE) and at least a mutation in nucleotide position 4595667 is detected with regard to reference genome NC_016822 as annotated at the NCBI, and
- wherein the antibiotic drug is CF and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is CFZ and a mutation in at least one of the following nucleotide positions is detected with regard to reference genome NC_016822 as annotated at the NCBI: 3134050; and/or
- wherein the antibiotic drug is A/S and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is CRM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is P/T and a mutation in at least one of the following nucleotide positions is detected with regard to reference genome NC_016822 as annotated at the NCBI: 2241215, 4595667, 4502174, 2543074, 1159191, 2325584, 4707429, 3134050, 1655745, 949314, 1427818, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is AM and a mutation in at least one of the following nucleotide positions is detected with regard to reference genome NC_016822 as annotated at the NCBI: 2241215, 2309519, 2314492, 4595667, 87512, 4502174, 2543074, 1159191, 2325584, 4707429, 3134050, 1655745, 949314, 1427818, 2636621, 1429397, 1690889, 4856482, 1723070, 4857482, 4705326, 862961, 2876708, 980321, 4429947, 4431976, 3376607, 4565385, 750613, 1781189, 4821412, 2533864, 1511130, 1513422, 1901825, 1990704, 2506615, 2539010, 2803779, 2877526, 3239464, 545532, 1257335, 906989, 3237959, 60014, 921872, 983367, 1182426, 1200860; and/or
- wherein the antibiotic drug is AUG and a mutation in at least one of the following nucleotide positions is detected with regard to reference genome NC_016822 as annotated at the NCBI: 2309519, 2314492, 4595667, 87512, 1159191, 2325584, 4707429, 3134050, 1427818, 2636621, 1429397, 2877526, 1200860; and/or
- wherein the antibiotic drug is TE and a mutation in at least one of the following nucleotide positions is detected with regard to reference genome NC_016822 as annotated at the NCBI: 4595667, 4707429, 1655745, 4705326, 2876708, 1990704, 2539010, 1257335.

3. The method of claim 1 or 2, wherein the resistance of a bacterial microorganism belonging to the species Shigella against 1, 2, 3, 4, 5, 6, 7, 8, 9, 10, 11, 12, 13, 14, 15 or 16, 17, 18, 19, 20 or 21 antibiotic drugs is determined.

4. The method of one or more of the preceding claims,
wherein determining the nucleic acid sequence information or the presence of a mutation comprises determining a partial sequence or an entire sequence of the at least two genes.

5. The method of one or more of the preceding claims,
wherein determining the nucleic acid sequence information or the presence of a mutation comprises determining a partial or entire sequence of the genome of the Shigella species, wherein said partial or entire sequence of the genome comprises at least a partial sequence of said at least two genes.

6. The method of one or more of the preceding claims,
wherein determining the nucleic acid sequence information or the presence of a mutation comprises using a next generation sequencing or high throughput sequencing method, preferably wherein a partial or entire genome sequence of the bacterial organism of Shigella species is determined by using a next generation sequencing or high throughput sequencing method.
